# EUROPÄISCHE PATENTSCHRIFT

(11) **EP 3 180 168 B1**
(45) Veröffentlichungstag und Bekanntmachung des Hinweises auf die Patenterteilung: **26.05.2021**
(21) Anmeldenummer: 15752920.7
(22) Anmeldetag: 13.08.2015
(51) Int. Cl.: B25J 9/00

(54) **POSITIONIERUNG EINES ROBOTERS**
POSITIONING A ROBOT
POSITIONNEMENT D'UN ROBOT

(30) Priorität: 14.08.2014 DE 102014012160; 14.08.2014 DE 102014012124
(43) Veröffentlichungstag der Anmeldung: 21.06.2017
(73) Patentinhaber: KUKA Deutschland GmbH, 86165 Augsburg (DE)
(72) Erfinder: BERGHOFER, Jakob, 80689 München (DE); BRUDNIOK, Sven, 86853 Langerringen (DE); LOHMEIER, Sebastian, 80639 München (DE); RIEDEL, Martin, 86165 Augsburg (DE)
(74) Vertreter: Schlotter, Alexander Carolus Paul
(86) Internationale Anmeldenummer: PCT/EP2015/001673
(87) Internationale Veröffentlichungsnummer: WO 2016/023635

(56) Entgegenhaltungen:
- EP-A2- 2 578 177
- DE-A1-102012 015 541
- US-A1- 2001 013 764
- US-A1- 2011 277 775

## Beschreibung

Die vorliegende Erfindung betrifft eine Vorrichtung und ein Verfahren zum Positionieren eines Roboters einer Roboteranordnung sowie eine Roboteranordnung mit einer solchen Vorrichtung.

Chirurgieroboter sind dazu vorgesehen, einen chirurgischen Endeffektor, beispielsweise ein Skalpell, eine Zange, Kamera oder Schlauchöffnung, am oder im Patienten relativ zu einer Chirurgieroboterbasis zu bewegen, indem Antriebe des Chirurgieroboters dessen Gelenke bewegen.

Nach betriebsinterner Praxis sind einerseits decken- oder bodenseitig montierte Chirurgieroboter bekannt, bei denen eine erste bzw. Basisdrehachse des Chirurgieroboters im Wesentlichen vertikal angeordnet ist. Andererseits sind auch wandseitig montierte Chirurgieroboter bekannt, bei denen die Basisdrehachse des Chirurgieroboters stattdessen im Wesentlichen horizontal angeordnet ist.

Insbesondere, um die Kinematik des Chirurgieroboters und/oder den verfügbaren Raum optimal zu nutzen und/oder Interferenzen mit weiteren Chirurgierobotern zu vermeiden, sind zudem nach betriebsinterner Praxis Positioniervorrichtungen bekannt, um die Chirurgieroboterbasis vor der Operation vor- und gegebenenfalls während der Operation umzupositionieren.

Die Bedienung einer solchen Positioniervorrichtung ist jedoch, insbesondere für medizinisches Personal ohne entsprechende kinematische Kenntnisse, schwierig. Die vorliegende Erfindung wird nachfolgend insbesondere mit Bezug auf einen Chirurgieroboter erläutert, für den die Erfindung besonders vorteilhaft ist. Sie kann jedoch auch mit Vorteil bei Industrie- und/oder Servicemanipulatoren, insbesondere - robotern, verwendet werden und ist daher nicht auf Chirurgieroboter beschränkt.

Aus der US 2001/0013764 A1 ist eine gattungsgemäße Positioniervorrichtung für einen Manipulator bekannt, die aufeinanderfolgend ein Schubgelenk, drei Drehgelenke mit parallelen Drehachsen und zwei weitere Drehgelenke aufweist, deren Drehachse jeweils senkrecht zur Drehachse des vorhergehenden Gelenks ist.

Die US 2011/0277775 A1 offenbart ebenfalls eine passive Positioniervorrichtung für einen Manipulator, die Schub- und Drehgelenke aufweist.

Aus der EP 2 578 177 A2 ist ein Chirurgierobotersystem mit Haupt- und Hilfsroboterarmen bekannt.

Eine Aufgabe der vorliegenden Erfindung ist es, das Positionieren eines Roboters, insbesondere eines Chirurgieroboters, zu verbessern.

Diese Aufgabe wird durch eine Positioniervorrichtung mit den Merkmalen des Anspruchs 1 bzw. ein Verfahren mit den Merkmalen des Anspruchs 14 gelöst. Anspruch 12 stellt eine entsprechende Roboter-, insbesondere Chirurgieroboteranordnung unter Schutz.

Nach einem Aspekt der vorliegenden Erfindung weist ein Trägersystem für einen Manipulator bzw. ein Trägersystem einer Manipulatoranordnung mit einem Manipulator einen Träger und einen Flansch auf, an dem der Manipulator, insbesondere lösbar, beispielsweise elektromagnetisch, hydraulisch und/oder durch Schrauben, befestigbar ist bzw. bzw. befestigt ist bzw. wird. Der Manipulator ist in einer Ausführung ein Roboter, insbesondere ein Chirurgie-, Industrie- oder Serviceroboter.

In einer Ausführung ist der Flansch fest mit dem Träger verbunden bzw. relativ zu diesem unbeweglich, insbesondere integral mit diesem ausgebildet. In einer anderen Ausführung ist der Flansch bzw. der daran befestigte Manipulator relativ zu dem Träger verstellbar, insbesondere ein- oder mehrachsig, insbesondere vertikal und/oder horizontal, verschieb- und/oder drehbar, beispielsweise durch eine Hubsäule und/oder ein Drehgelenk.

In einer Ausführung ist der Flansch des Trägersystems der Positioniervorrichtungsflansch einer nachfolgend näher erläuterten Positioniervorrichtung nach einem weiteren Aspekt der vorliegenden Erfindung und der Träger des Trägersystems der Träger dieser Positioniervorrichtung, an dem deren Positioniervorrichtungsbasis ein- oder mehrachsig linear beweglich und/oder drehbar gelagert ist, wobei der Manipulator bzw. Chirurgieroboter an dem Positioniervorrichtungsflansch der Positioniervorrichtung befestigbar ist bzw. befestigt ist bzw. wird. Entsprechend kann in einer Ausführung die nachfolgend erläuterte Positioniervorrichtung insbesondere das hier beschriebene Trägersystem aufweisen. Wie einleitend erläutert, kann dieses jedoch gleichermaßen auch unabhängig hiervor eingesetzt werden, insbesondere für bzw. mit einem Industrie- oder Servicemanipulator, insbesondere -roboter.

Nach einem Aspekt der vorliegenden Erfindung weist das Trägersystem eine an dem Träger angeordnete, insbesondere lösbar oder dauerhaft mit dem Träger verbundene, Luftkammeranordnung mit einem oder mehreren Druckräumen auf, die von einer Druckschürze umkleidet sind.

Nach einem Aspekt ist die Luftkammeranordnung, insbesondere einer oder mehrere ihrer Druckräume, zur Ausbildung eines ein- oder mehrzelligen Luftkissens mit einer Überdruckluftversorgung verbindbar, insbesondere ist bzw. wird die Luftkammeranordnung, insbesondere einer oder mehrere ihrer Druckräume, zur Ausbildung eines ein- oder mehrzelligen Luftkissens mit einer Überdruckluftversorgung verbunden.

Hierdurch kann das Trägersystem, insbesondere mit dem an dem Flansch befestigten Manipulator oder, beispielsweise für ein Wechseln des Manipulators, auch ohne einen an dem Flansch befestigten Manipulator, vorteilhaft, insbesondere manuell, bewegt werden, insbesondere präzise, geräusch-, reibungs- und/oder abriebsarm und/oder über Hindernisse wie beispielsweise Schläuche, Schwellen oder dergleichen.

In einer Ausführung umkleidet eine gemeinsame Druckschürze zwei oder mehr, insbesondere alle Druckräume der Luftkammeranordnung. Hierdurch kann eine kompakte und/oder günstige Bauweise erreicht werden. In einer Ausführung weist die Luftkammeranordnung wenigstens zwei Druckschürzen auf, die jeweils einen oder mehrere Druckräume der Luftkammeranordnung umkleiden, bzw. sind wenigstens zwei Druckräume durch verschiedene Druckschürzen umkleidet. Hierdurch kann vorteilhaft eine erhöhte Ausfallsicherheit und/oder Austauschbarkeit erreicht werden.

In einer Ausführung umkleidet eine Druckschürze einen Druckraum umfangsseitig, dieser ist somit zum Untergrund hin offen. In einer Ausführung ist eine Druckschürze flexibel, beispielsweise aus Gummi oder, insbesondere beschichtetem, Textilmaterial oder einem elastischen Kunststoff. Entsprechend weist ein Druckraum in einer Ausführung ein variables Volumen auf, das vorzugsweise zum Bewegen des Trägers bzw. zur Ausbildung des Luftkissens vergrößert und/oder zum Fixieren des Trägers mittels Unterdruck verkleinert wird. Der Druckraum wird in einer Ausführung entsprechend umfangsseitig von der Druckschürze und deckenseitig von einem Boden des Trägers und/oder einem daran angeordneten Teil der Druckschürze begrenzt bzw. gebildet.

Insbesondere bei einem den Träger, insbesondere mit dem damit verbundenen Manipulator, tragenden ein- oder mehrzelligen Luftkissen, das durch den bzw. die mit der Überdruckluftversorgung verbundenen Druckräume ausgebildet ist bzw. wird, können durch die Überdruckluft, die durch einen Spalt zwischen Boden und Druckschürze(n) austritt, ein störendes Geräusch entstehen und/oder Partikel, insbesondere Staub, ver-, insbesondere vom Boden aufgewirbelt werden. Gleichermaßen können bei einem den Träger fixierenden Unterdruck Geräusche durch in den bzw. die Druckräume eingesaugte Umgebungsluft entstehen. Dies ist insbesondere bei einem Einsatz als Trägersystem einer Chirurgieroboteranordnung nachteilig.

Daher weist die Luftkammeranordnung in einer Ausführung wenigstens einen von einer Schallschürze umkleideten Schallraum auf, der wenigstens einen der Druckräume, insbesondere umfangsseitig, umgibt.

In einer Ausführung umkleidet eine gemeinsame Schallschürze zwei oder mehr, insbesondere alle Druckräume bzw. -schürzen der Luftkammeranordnung, vorzugsweise konzentrisch. Hierdurch kann eine kompakte und/oder günstige Bauweise erreicht werden. In einer Ausführung weist die Luftkammeranordnung wenigstens zwei Schallschürzen auf, die jeweils einen oder mehrere Druckräume bzw. -schürzen der Luftkammeranordnung umkleiden, vorzugsweise konzentrisch, bzw. sind wenigstens zwei Druckräume bzw. -schürzen durch verschiedene Schallschürzen bzw. -räume, vorzugsweise konzentrisch, umkleidet. Hierdurch kann vorteilhaft eine erhöhte Ausfallsicherheit erreicht werden.

In einer Ausführung umkleidet eine Schallschürze eine oder mehrere Druckschürzen bzw. -räume umfangsseitig, der entsprechende Schallraum ist somit zum Untergrund hin offen. In einer Ausführung ist eine Schallschürze flexibel, beispielsweise aus Gummi oder, insbesondere beschichtetem, Textilmaterial oder einem elastischen ·Kunststoff. Entsprechend weist ein Schallraum in einer Ausführung ebenfalls ein variables Volumen auf, das sich mit dem Volumen des bzw. der Druckräume vergrößert bzw. -kleinert. Ein Schallraum wird in einer Ausführung entsprechend umfangsseitig innen von der bzw. den Druckschürzen, außen von der Schallschürze und deckenseitig von einem Boden des Trägers und/oder einem daran angeordneten Teil der Druck- oder Schallschürze(n) begrenzt bzw. gebildet.

In einer Ausführung sind wenigstens eine Schall- und Druckschürze integral miteinander ausgebildet, was die Herstellung und/oder Montage verbessern kann. In einer anderen Ausführung sind wenigstens eine Schall- und Druckschürze separat voneinander ausgebildet, was die Auslegung und/oder den Ersatz verbessern kann.

In einer Ausführung sitzt eine Druck- und/oder Schallschürze bei einem Bewegen des Trägers und/oder bei fixiertem Träger dichtend auf dem Boden bzw. Untergrund auf bzw. ist hierzu vorgesehen bzw. eingerichtet. Hierzu kann eine Schürze insbesondere ein, vorzugsweise flexibles, bodenseitiges Dichtelement, beispielsweise eine Dichtlippe oder dergleichen, aufweisen. Zusätzlich oder alternativ kann die Schürze gegen den Boden bzw. Untergrund, insbesondere elastisch, vorgespannt sein bzw. werden.

Eine Druck- und/oder Schallschürze ist in einer Ausführung durch eine oder mehrere konzentrische Reihen über den Umfang verteilter, insbesondere einander überlappender, Lamellen ausgebildet. Gleichermaßen kann eine Druck- und/oder Schallschürze beispielsweise auch durch eine ein- oder mehrlagige geschlossene Wandung ausgebildet sein.

In einer Weiterbildung weist das Trägersystem wenigstens ein Schalldämpfermittel zum Dämpfen von Luftschall in wenigstens einem Schallraum auf. Das Schalldämpfermittel kann insbesondere einen oder mehrere Reflexionsschalldämpfer mit mehreren, insbesondere wenigstens vier, miteinander, insbesondere mit Querschnittserweiterungen und/oder -verengungen, kommunizierenden Kammern, in denen in einer Ausführung, insbesondere gegeneinander geneigte Prallwände angeordnet sind, und/oder einen oder mehrere, insbesondere lamellenartige, Absorptionsschalldämpfer, vorzugsweise mit poröses Material, insbesondere Flies, Gewebe, Steinwolle, Glaswolle und/oder Glasfaser, aufweisen.

Hierdurch werden vorteilhaft Schwingungen der Überdruckluft, die zur Ausbildung des Luftkissens aus einem Spalt zwischen Druckschürze(n) und Boden in einen Schallraum austritt, und/oder Schwingungen von Umgebungsluft, die infolge eines den Träger fixierenden Unterdrucks eingesaugt wird, gedämpft und so eine Geräuschemission und/oder Partikelverwirbelung reduziert.

In einer Weiterbildung ist ein Schalldämpfermittel in einem Schallraum angeordnet. Hierdurch kann Luftschall und/oder Partikelaufwirbelung vorteilhaft nahe am Entstehungsort reduziert werden. Insbesondere können in einem Schallraum eine oder mehrere konzentrische Lamellenreihen angeordnet sein, die vorzugsweise im Betrieb, insbesondere bei ausgebildetem Luftkissen auf dem Boden schleifen und/oder schallabsorbierendes Material wie beispielsweise textile Fliese, Gewebe oder dergleichen aufweisen. Zusätzlich oder alternativ kann auch die Schallschürze selber durch solche Lamellen ausgebildet sein.

Zusätzlich oder alternativ kann ein Schalldämpfermittel von einem Schallraum beabstandet sein und mit diesem Schallraum, insbesondere über eine Luftpassage, insbesondere -leitung, kommunizieren. Hierdurch kann das Schalldämpfermittel an einem vorteilhaften Ort angeordnet werden bzw. sein.

Ein solches von einem Schallraum beabstandetes und mit diesem kommunizierendes Schalldämpfermittel kann - wie ein in dem Schallraum angeordnetes Schalldämpfermittel - trägerfest bzw. -seitig sein. So kann beispielsweise Überdruckluft, die durch einem Spalt zwischen Boden und Druckschürze aus einem Druck- in einen Schallraum austritt, in dem Schallraum schallabsorbierende Lamellen durch- bzw. umströmen, bevor es durch einen Spalt zwischen Boden und der vorzugsweise ebenfalls als Lamelle ausgebildete Schallschürze gedämpft in die Umgebung austritt. Gleichermaßen kann die Luft aus dem Schallraum in ein trägerfestes Schalldämpfermittel abgeleitet werden, aus der sie bodennah oder bodenfern austreten kann, um eine Aufwirbelung von Bodenstaub zu reduzieren.

In einer anderen Ausführung ist ein von einem Schallraum beabstandetes und mit diesem kommunizierendes Schalldämpfermittel ein trägerfernes bzw. -externes Schalldämpfermittel, das mit dem Schallraum vorzugsweise über eine von dem Träger abgehende Luftleitung kommuniziert. Das trägerexterne Schalldämpfermittel kann so die Luft an einer von dem Träger entfernten Stelle in die Umgebung ausleiten. In einer Ausführung ist ein solches trägerexternes Schalldämpfermittel stationär bzw. umgebungsfest, in einer anderen Ausführung seinerseits ebenfalls beweglich bzw. mobil.

Nach einem Aspekt der vorliegenden Erfindung werden zum Bewegen des Trägers ein oder mehrere Druckräume der Luftkammeranordnung von einer mit dieser verbundenen Überdruckluftversorgung versorgt bzw. mit Luft unter Überdruck beschickt und der hierdurch luftkissengetragene Träger, insbesondere manuell oder motorisch, bewegt.

Um den Träger sicher zu fixieren, wird nach einem weiteren Aspekt der vorliegenden Erfindung, der mit dem hier beschriebenen Luftkissenaspekt kombiniert sein kann, in einem oder mehreren Druckräumen der Luftkammeranordnung durch eine damit verbundene Unterdruckluftversorgung, insbesondere satt eines Luftkissens, ein den Träger fixierender Unterdruck ausgebildet. Entsprechend weist das Trägersystem nach einem Aspekt der vorliegenden Erfindung eine mit einem oder mehreren Druckräumen der Luftkammeranordnung verbindbare, insbesondere verbundene, Überdruckluftversorgung zur Versorgung mit Überdruckluft und/oder eine mit einem oder mehreren, insbesondere denselben oder anderen, Druckräumen der Luftkammeranordnung verbindbare, insbesondere verbundene, Unterdruckluftversorgung zur Ausbildung eines den Träger fixierenden Unterdrucks auf.

Dabei können, wie vorstehend angedeutet, ein oder mehrere Druckräume der Luftkammeranordnung in einer Ausführung wahlweise mit der Über- oder Unterdruckversorgung verbunden werden, um alternativ eine Zelle eines Luftkissens oder einen den Träger am Untergrund fixierenden Unterdruck auszubilden. Hierdurch kann vorteilhaft die Luftkammeranordnung kompakter ausgebildet werden. Gleichermaßen kann die Luftkammeranordnung auch einen oder mehrere Druckräume aufweisen, die nur mit der Überdruckluftversorgung verbindbar, insbesondere verbunden sind bzw. werden, und einen oder mehrere hiervon verschiedene Druckräume, die nur mit der Unterdruckluftversorgung verbindbar, insbesondere verbunden sind bzw. werden. Hierdurch kann vorteilhaft der Träger luftkissengetragen am Untergrund fixiert werden.

Gleichermaßen kann, wie ebenfalls bereits erläutert, die Luftkammeranordnung nach einem Aspekt auch nur mit einer Überdruckluftversorgung zur Ausbildung eines Luftkissens oder nur mit einer Unterdruckluftversorgung zur Fixierung am Untergrund verbindbar sein, insbesondere verbunden sein bzw. werden. Beispielsweise kann auch ein rad-, rollen-, schienen- oder gleitbeweglicher Träger durch Unterdruck in der Luftkammeranordnung am Untergrund fixierbar sein, insbesondere fixiert sein bzw. werden. Gleichwohl stellt die wahlweise Nutzung der Luftkammeranordnung als Luftkissen oder zur Fixierung eine besonders vorteilhafte Doppelnutzung dar.

Die Überdruckluftversorgung und/oder Unterdruckluftversorgung weist in einer Ausführung einen trägerfesten bzw. -seitigen Luftleitungsanschluss auf, der mit einer trägerfernen bzw. externen, insbesondere stationären oder ihrerseits mobilen, Überdruckluftquelle bzw. Unterdruckluftquelle verbindbar ist, insbesondere verbunden ist bzw. wird. Insbesondere kann eine Über- und/oder Unterdruckluftversorgung im Sinne der vorliegenden Erfindung ein solcher trägerfester bzw. -seitiger Luftleitungsanschluss, vorzugsweise in Verbindung mit der trägerfernen bzw. externen Über- bzw. Unterdruckluftquelle sein. Hierdurch kann der Träger entlastet werden. Zusätzlich oder alternativ kann die Überdruckluftversorgung und/oder die Unterdruckluftversorgung eine trägerfeste bzw. -seitige Überdruckluftquelle bzw. Unterdruckluftquelle aufweisen. Hierdurch kann der Träger, wenigstens zeitweise, autonom betrieben werden.

Eine trägerseitige und/oder eine trägerexterne Überdruckluftquelle weist in einer Ausführung einen Druckspeicher auf, in dem Luft unter Überdruck gegenüber der Umgebung ("Überdruckluft") gespeichert ist. Entsprechend kann eine trägerseitige und/oder eine trägerexterne Unterdruckluftquelle in einer Ausführung einen Druckspeicher aufweisen, in dem Unterdruck gegenüber der Umgebung herrscht. Zusätzlich oder alternativ weist in einer Ausführung eine trägerseitige und/oder eine trägerexterne Überdruckluftquelle eine Strömungsmaschine zur Druckerhöhung von durch die Strömungsmaschine angesaugter Luft, insbesondere Umgebungsluft, auf, insbesondere einen Kompressor. Entsprechend kann eine trägerseitige und/oder eine trägerexterne Unterdruckluftquelle in einer Ausführung einen Strömungsmaschine zur Druckreduzierung von durch die Strömungsmaschine angesaugter Luft, insbesondere Umgebungsluft oder Luft aus der Luftkammeranordnung, aufweisen, insbesondere einen Kompressor zum Absaugen von Luft aus der Luftkammeranordnung oder eine mit einem Kompressor verbundene Venturidüse zum Absaugen von Luft aus der Luftkammeranordnung.

Entsprechend werden vorliegend zur kompakteren Darstellung ein Druckspeicher, in dem Unterdruck gegenüber der Umgebung herrscht, sowie eine Strömungsmaschine zum Erzeugen von Unterdruck gegenüber der Umgebung als (Unter)Druckluftquelle bzw. -versorgung bezeichnet. Zur kompakteren Darstellung werden sowohl eine Über- als auch eine Unterdruckluftversorgung allgemein als Druckluftversorgung, eine Über- und eine Unterdruckluftquelle allgemein als Druckluftquelle bezeichnet.

Die Über- und Unterdruckluftversorgung kann in einer Ausführung denselben Luftleitungsanschluss zur wahlweisen bzw. alternativen Verbindung mit einer trägerexternen Über- bzw. Unterdruckluftquelle, und/oder dieselbe, wahlweise gegensinnig betriebene Strömungsmaschine aufweisen. Hierdurch kann die Druckluftversorgung kompakter ausgebildet werden. Gleichermaßen können die Über- und Unterdruckluftversorgung in einer Ausführung unterschiedliche Luftleitungsanschlüsse zur, insbesondere parallelen, Verbindung mit einer trägerexternen Über- bzw. Unterdruckluftquelle, und/oder zwei Strömungsmaschinen aufweisen. Hierdurch können vorteilhaft die Druckluftversorgungen optimiert und/oder parallel genutzt werden.

In einer Ausführung weist das Trägersystem eine Rollenanordnung mit zwei oder mehr Transportrollen zum Verfahren des Trägers auf. Hierdurch kann insbesondere bei einem Nachlassen oder Versagen des Luftkissens der Träger weiterhin bewegt werden. Die Rollenanordnung weist in einer Weiterbildung drei oder vier Transportrollen auf.

Bei ausgebildetem Luftkissen ist bzw. wird die Rollenanordnung vorzugsweise vom Boden beabstandet, um die Vorteile des tragenden Luftkissens voll auszunutzen. Hierzu weisen in einer Ausführung das Luftkissen bzw. die Druckschürze(n) bei ausgebildetem Luftkissen eine vertikale Erstreckung auf, die größer ist als die vertikale Erstreckung der Rollenanordnung, so dass diese durch das ausgebildete Luftkissen vom Boden abgehoben wird bzw. ist.

Zusätzlich oder alternativ kann die Rollenanordnung in einer Ausführung, insbesondere selbsttätig, automatisch und/oder manuell, von einer Betriebs- in eine Lagerstellung verstellt, insbesondere verschwenkt oder eingefahren werden, vorzugsweise durch oder nach Ausbilden des Luftkissens und/oder durch oder vor Ausbilden des Unterdrucks. Zusätzlich oder alternativ kann sie, insbesondere selbsttätig, automatisch und/oder manuell, von der Lager- in die Betriebsstellung verstellt, insbesondere verschwenkt oder ausgefahren werden, insbesondere durch oder vor Abbau des Luftkissens und/oder durch oder nach Abbau des Unterdrucks.

Zusätzlich oder alternativ kann die Rollenanordnung in einer Ausführung lösbar mit dem Träger verbindbar, insbesondere verbunden sein bzw. werden. In einer anderen Ausführung ist die Rollenanordnung hingegen stationär bzw. dauerhaft an dem Träger angeordnet.

In einer Ausführung ist die Rollenanordnung in einem oder mehreren der Druckräume angeordnet, was eine besonders platzsparende und statisch vorteilhafte Abstützung ermöglicht.

In einer Ausführung weist das Trägersystem eine Stützanordnung mit zwei oder mehr, insbesondere elastischen, Stützen zur Abstützung des Trägers, insbesondere anstelle des Luftkissens, auf. Wie vorstehend mit Bezug auf die Rollenanordnung erläutert, zu der die Stützanordnung alternativ oder zusätzlich vorgesehen sein kann, ist bei ausgebildetem Luftkissen die Stützanordnung vorzugsweise vom Boden beabstandet. Hierzu weisen in einer Ausführung das Luftkissen bzw. die Druckschürze(n) bei ausgebildetem Luftkissen eine vertikale Erstreckung auf, die größer ist als die vertikale Erstreckung der Stützanordnung, so dass diese durch das ausgebildete Luftkissen vom Boden abgehoben wird bzw. ist. Der durch Unterdruck fixierte Träger stützt sich in einer Ausführung auf der Rollen- und/oder Stützanordnung ab.

Zusätzlich oder alternativ kann die Stützanordnung in einer Ausführung, insbesondere, selbsttätig, automatisch und/oder manuell, von einer Betriebs- in eine Lagerstellung verstellt, insbesondere verschwenkt oder eingefahren werden, vorzugsweise durch oder nach Ausbilden des Luftkissens. Zusätzlich oder alternativ kann sie, insbesondere selbsttätig, automatisch und/oder manuell, von der Lager- in die Betriebsstellung verstellt, insbesondere verschwenkt oder ausgefahren werden, werden, insbesondere durch oder vor Abbau des Luftkissens und/oder durch oder vor Ausbilden des Unterdrucks.

Zusätzlich oder alternativ kann die Stützanordnung in einer Ausführung lösbar mit dem Träger verbindbar, insbesondere verbunden sein bzw. werden. In einer anderen Ausführung ist die Stützanordnung hingegen stationär bzw. dauerhaft an dem Träger angeordnet.

In einer Ausführung weist das Trägersystem eine Steuerung und/oder eine, insbesondere damit kommunizierende, Bedieneinrichtung zur, insbesondere geregelten, Versorgung eines oder mehrerer Druckräume mit Überdruckluft durch die Überdruckluftversorgung und/oder zur, insbesondere alternativen, Versorgung mit Unterdruck(luft) durch die Unterdruckluftversorgung auf.

Die Bedieneinrichtung ist in einer Ausführung an dem Träger, insbesondere einem Handgriff zum Bewegen des Trägers, angeordnet, beispielsweise in Form eines oder mehrerer Betätigungsschalter, -knöpfe oder dergleichen. Hierdurch kann vorteilhaft das Luftkissen beim Bewegen des Trägers bzw. der Unterdruck beim Fixieren des Trägers gesteuert, insbesondere aktiviert und/oder deaktiviert werden. Zusätzlich oder alternativ kann Bedieneinrichtung auch eine Fernbedieneinrichtung zum von dem Träger beabstandeten Steuern des Luftkissens und/oder Unterdrucks sein, die in einer Weiterbildung lösbar mit dem Träger verbindbar, insbesondere verbunden ist bzw. wird.

Die Steuerung weist in einer Ausführung ein oder mehrere, insbesondere stufenlos verstellbare und/oder elektrisch betätigte, Steuer- bzw. Stellventile zum wahlweisen Versorgen von einem oder mehreren Druckräumen mit Überdruckluft oder/oder Unterdruck durch die Über- bzw. Unterdruckluftversorgung und/oder einen Controller zum Ansteuern dieser Ventile und/oder einer Strömungsmaschine der Über- und/oder Unterdruckluftversorgung auf. In einer Weiterbildung ist der Controller, insbesondere software- bzw. programtechnisch, dazu eingerichtet, einen oder mehrere Druckräume, insbesondere gemeinsam oder unabhängig voneinander, mit Überdruckluft und/oder Unterdruck durch die Über- bzw. Unterdruckversorgung zu versorgen. In einer Weiterbildung ist der Controller, insbesondere software- bzw. programtechnisch, dazu eingerichtet, den Über- und/oder Unterdruck in dem bzw. Druckräumen auf ein vorgegebenes Niveau zu regeln, indem er diesen geregelt bzw. auf Basis eines erfassten Ist-und eines vorgegebenen Soll-Zustandes, beispielsweise Luftdrucks oder vertikalen Abstandes des Trägers zum Boden, mit Überdruckluft bzw. Unterdruck durch die Über- bzw. Unterdruckversorgung versorgt.

In einer Ausführung regelt die Steuerung eine Versorgung der Luftkammeranordnung, insbesondere einer oder mehrerer ihrer Druckkammern, mit Überdruckluft und/oder Unterdruck durch die mit ihr verbundene Über- und/oder Unterdruckluftversorgung, insbesondere eine Luftzu- und/-der -abfuhr bzw. einen Über- und/oder Unterdruck in einer oder mehreren Druckkammern der Luftkammeranordnung, auf Basis eines Kippmoments des Trägersystems um die Horizontale bzw. ist hierzu eingerichtet.

In einer Ausführung erhöht die Steuerung einen Über- oder Unterdruck in einer oder mehreren der Luftkammern der Luftkammeranordnung, wenn ein Kippmoment des Trägersystems um die Horizontale sich vergrößert, und/oder reduziert einen Über- oder Unterdruck in einer oder mehreren der Luftkammern der Luftkammeranordnung, wenn ein Kippmoment des Trägersystems um die Horizontale sich verringert, bzw. ist hierzu eingerichtet.

In einer Ausführung erhöht die Steuerung einen Überdruck in einer oder mehreren der Luftkammern derart, dass dieser ein Gegenmoment zu dem Kippmoment induziert, also insbesondere in Luftkammern auf einer Seite des Trägers, auf die das Kippmoment das Trägersystem zu kippen sucht, und/oder erhöht einen Unterdruck in einer oder mehreren der Luftkammern derart, dass dieser ein Gegenmoment zu dem Kippmoment induziert, also insbesondere in Luftkammern auf einer Seite des Trägers, die das Kippmoment das Trägersystem anzuheben sucht, bzw. ist hierzu eingerichtet.

Gleichermaßen kann die Steuerung in allen Luftkammern den Über- und/oder Unterdruck erhöhen, wenn ein Kippmoment des Trägersystems um die Horizontale sich vergrößert, und/oder den Über- oder Unterdruck in allen Luftkammern der Luftkammeranordnung reduzieren, wenn ein Kippmoment des Trägersystems um die Horizontale sich verringert, bzw. hierzu eingerichtet sein.

Ein Kippmoment des Trägersystems um die Horizontale, d.h. ein Drehmoment, das auf das Trägersystem wirkt und dieses um eine horizonatle Achse zu drehen bzw. kippen sucht, hängt insbesondere von einer Pose eines an dem Träger befestigten Manipulators ab: weist dieser eine große Auslage und einen entsprechend von dem Schwerpunkt des Trägersystems beabstandeten Schwerpunkt auf, induziert dies ein Kippmoment.Daher regelt in einer Ausführung die Steuerung die Versorgung der Luftkammeranordnung mit Überdruckluft und/oder Unterdruck auf Basis einer Pose des an dem Trägersystem befestigten Manipulators bzw. ist hierzu eingerichtet. In einer Ausführung erhöht die Steuerung einen Über- oder Unterdruck in einer oder mehreren der Luftkammern der Luftkammeranordnung, wenn eine Auslage des Manipulators sich vergrößert, und/oder reduziert einen Über- oder Unterdruck in einer oder mehreren der Luftkammern der Luftkammeranordnung, wenn eine Auslage des Manipulators sich verringert, bzw. ist hierzu eingerichtet. In einer Ausführung erhöht die Steuerung einen Überdruck in einer oder mehreren Luftkammern auf einer der Seite des Trägers, zu der der Manipulator eine Auslage aufweist, und/oder erhöht einen Unterdruck in einer oder mehreren Luftkammern auf einer der Auslage gegenüberligenenden Seite bzw. ist hierzu eingerichtet. Gleichermaßen kann die Steuerung in allen Luftkammern den Über- und/oder Unterdruck erhöhen, wenn eine Auslage des Manipulator sich vergrößert, und/oder den Über- oder Unterdruck in allen Luftkammern der Luftkammeranordnung reduzieren, wenn eine Auslage des Manipulator sich verringert, bzw. hierzu eingerichtet sein.

Unter einer Auslage wird vorliegend insbesondere ein Abstand eines Schwerpunktes bzw. Massenmittelpunktes des Manipulators von einer, vorzugsweise statisch stabilen, insbesondere kippmomentfreien, Referenz, verstanden. Gleichermaßen kann eine Auslage beispielsweise auch einen horizontalen Abstand zwischen einem TCP und einer Basis des Manipulators bezeichnen. Die Auslage kann aus der Pose des Manipulators ermittelt werden. Entsprechend weist das Trägersystem in einer Ausführung ein Erfassungsmittel zum Erfassen einer Pose des Manipulators auf, das mit der Steuerung signalverbunden ist, beispielsweise Gelenksensoren oder dergleichen.

Ein Kippmoment des Trägersystems um die Horizontale hängt insbesondere von einer Traglast eines an dem Träger befestigten Manipulators ab: weist dieser eine größere Traglast auf, kann dies ein größeres Kippmoment induzieren.Daher regelt in einer Ausführung die Steuerung die Versorgung der Luftkammeranordnung mit Überdruckluft und/oder Unterdruck auf Basis einer Tragslast des an dem Trägersystem befestigten Manipulators bzw. ist hierzu eingerichtet. In einer Ausführung erhöht die Steuerung einen Über- oder Unterdruck in einer oder mehreren der Luftkammern der Luftkammeranordnung, wenn eine Traglast des Manipulators sich vergrößert, und/oder reduziert einen Über- oder Unterdruck in einer oder mehreren der Luftkammern der Luftkammeranordnung, wenn eine Traglast des Manipulators sich verringert, bzw. ist hierzu eingerichtet.

Unter einer Traglast wird vorliegend insbesondere eine am Manipulator, vorzugsweise lösbar, befestigte Nutzlast verstanden. Sie kann beispielsweise aus Antriebskräften des Manipulators ermittelt werden. Entsprechend weist das Trägersystem in einer Ausführung ein Erfassungsmittel zum Erfassen einer Traglast des Manipulators auf, das mit der Steuerung signalverbunden ist, beispielsweise Gelenkmomentsensoren oder dergleichen.

Ein Kippmoment des Trägersystems um die Horizontale beeinflusst insbesondere eine Lage des Trägers: ein größeres Kippmoment bewirkt eine stärkere Auslenkung des Trägers gegen die Horizontale. Daher regelt in einer Ausführung die Steuerung die Versorgung der Luftkammeranordnung mit Überdruckluft und/oder Unterdruck auf Basis einer Lage des Trägers bzw. ist hierzu eingerichtet. In einer Ausführung erhöht die Steuerung einen Über- oder Unterdruck in einer oder mehreren der Luftkammern der Luftkammeranordnung, wenn eine Auslenkung des Trägers gegen die Horizontale sich vergrößert, und/oder reduziert einen Über- oder Unterdruck in einer oder mehreren der Luftkammern der Luftkammeranordnung, wenn eine Auslenkung des Trägers gegen die Horizontale sich verringert, bzw. ist hierzu eingerichtet. In einer Ausführung erhöht die Steuerung einen Überdruck in einer oder mehreren Luftkammern auf einer der Seite des Trägers, zu der der Träger sich neigt, und/oder erhöht einen Unterdruck in einer oder mehreren Luftkammern auf einer Seite, von der der Träger sich wegneigt, bzw. ist hierzu eingerichtet. Gleichermaßen kann die Steuerung in allen Luftkammern den Über- und/oder Unterdruck erhöhen, wenn eine Auslenkung des Trägers gegen die Horizontale sich vergrößert, und/oder den Über- oder Unterdruck in allen Luftkammern der Luftkammeranordnung reduzieren, wenn eine Auslenkung des Trägers gegen die Horizontale sich verringert, bzw. hierzu eingerichtet sein.

Entsprechend weist das Trägersystem in einer Ausführung ein Erfassungsmittel zum Erfassen einer Lage, insbesondere Auslenkung gegen die Horizontale, des Trägers auf, das mit der Steuerung signalverbunden ist, beispielsweise ein oder mehrere voneinander beabstandete Abstandssensoren zur Erfassung eines vertikalen Bodenabstandes des Trägers, Winkellagesensoren oder dergleichen.

Ein Kippmoment des Trägersystems um die Horizontale und ein Luftdruck in der Luftkammeranordnung hängen wechsekseitig voneinander ab: so kann ein Kippmoment den Überdruck in Luftkammern, die diesem entgegenwirken, erhöhen und/oder durch Vergrößerung eines Bodenspaltes einen Unterdruck in Luftkammern reduzieren, die dem Kippmoment entgegenwirken. Umgekehrt bewirkt ein Absinken eines Überdrucks oder eine Vergrößerung eines Unterdrucks in Luftkammern ein Kippmoment zur Seite dieser Kammern. Daher regelt in einer Ausführung die Steuerung die Versorgung der Luftkammeranordnung mit Überdruckluft und/oder Unterdruck auf Basis eines Luftdrucks in der Luftkammeranordnung bzw. ist hierzu eingerichtet. In einer Ausführung erhöht die Steuerung eine Luftzufuhr zu einer oder mehreren der Luftkammern der Luftkammeranordnung, wenn ein Überdruck in einer oder mehreren der Luftkammern der Luftkammeranordnung sich verringert, erhöht eine Luftabfuhr aus einer oder mehreren der Luftkammern der Luftkammeranordnung, wenn ein Unterdruck in einer oder mehreren der Luftkammern der Luftkammeranordnung sich verringert, reduziert eine Luftzufuhr zu einer oder mehreren der Luftkammern der Luftkammeranordnung, wenn ein Überdruck in einer oder mehreren der Luftkammern der Luftkammeranordnung sich vergrößert, und/oder reduziert eine Luftabfuhr aus einer oder mehreren der Luftkammern der Luftkammeranordnung, wenn ein Unterdruck in einer oder mehreren der Luftkammern der Luftkammeranordnung sich vergrößert bzw. ist hierzu eingerichtet.

Entsprechend weist das Trägersystem in einer Ausführung ein Erfassungsmittel zum Erfassen eines Luftdrucks in der Luftkammeranordnung auf, das mit der Steuerung signalverbunden ist, beispielsweise Drucksensoren oder dergleichen.

Nach einem Aspekt der vorliegenden Erfindung weist eine Positioniervorrichtung für einen Roboter, insbesondere Chirurgieroboter bzw. eine Positioniervorrichtung einer Roboter-, insbesondere Chirurgieroboteranordnung mit einem Roboter, insbesondere Chirurgieroboter eine (Positioniervorrichtungs)Basis und einen (Positioniervorrichtungs)Flansch auf, an dem der Roboter, insbesondere Chirurgieroboter, insbesondere lösbar, beispielsweise elektromagnetisch, hydraulisch und/oder durch Schrauben, befestigbar bzw. befestigt ist bzw. wird.

Der Roboter weist in einer Ausführung einen, insbesondere chirurgischen, Endeffektor auf, der in einer Weiterbildung zum mechanischen, optischen und/oder fluidischen Arbeiten, insbesondere an oder in einem Patienten, vorgesehen bzw. eingerichtet ist, insbesondere zum Pressen, insbesondere Klemmen, Trennen, insbesondere Schneiden oder Stechen, Bohren, Aussenden und/oder Auffangen von elektromagnetischer Strahlung, insbesondere sichtbarem Licht, und/oder Ein- und/oder Ausströmen von Flüssigkeiten und/oder Gasen. Der Endeffektor ist bzw. wird in einer Ausführung, insbesondere über einen Instrumentenschaft, lösbar an einem Roboterflansch des Roboters befestigbar bzw. befestigt, beispielsweise elektromagnetisch, hydraulisch und/oder durch Verschrauben, -rasten oder Klemmen. Der Instrumentenschaft ist bzw. wird in einer Ausführung in eine natürliche oder künstliche Körperöffnung eingeführt bzw. ist hierzu vorgesehen bzw. eingerichtet, deren Umfang in einer Weiterbildung höchstens das Doppelte eines maximalen Umfangs des Endeffektors und/oder höchstens 15 cm beträgt. Somit ist der Chirurgieroboter in einer Ausführung für die minimalinvasive Chirurgie vorgesehen bzw. eingerichtet.

In einer Ausführung weist der Roboter wenigstens sechs, insbesondere wenigstens sieben, vorzugsweise durch, insbesondere elektromotorische, Antriebe des Roboters bewegbare, Gelenke, insbesondere Drehgelenke, auf, die den Endeffektor bzw. Roboterflansch mit einer Roboterbasis verbinden, die an dem Positioniervorrichtungsflansch befestigbar bzw. befestigt ist bzw. wird.

Der Positioniervorrichtungsflansch ist durch eine Kinematik, die wenigstens zwei Gelenke, insbesondere Drehgelenke aufweist, mit der Positioniervorrichtungsbasis verbunden. Durch diese Kinematik ist bzw. wird der Positioniervorrichtungsflansch, insbesondere translatorisch, von einer, insbesondere kartesischen, ersten Position relativ zu der Positioniervorrichtungsbasis, insbesondere wenigstens im Wesentlichen auf einer Kreisbahn oder Geraden, in eine hiervon beabstandete, insbesondere kartesische, zweite Position relativ zu der Positioniervorrichtungsbasis verstellbar bzw. verstellt. Die Gelenke der Kinematik sind durch, vorzugsweise starre, Glieder, insbesondere Hebel wie Stangen, Balken, Schalen oder dergleichen, miteinander verbunden, die entsprechend als Glieder der Kinematik bezeichnet werden. Diese werden zusammen mit dem Positioniervorrichtungsflansch und der Positioniervorrichtungsbasis gemeinsam als Glieder der Positioniervorrichtung bezeichnet.

In einer Ausführung ist bzw. wird der Positioniervorrichtungsflansch vor einem Operationsbetrieb, insbesondere vor einer (initialen) Interaktion des Endeffektors mit dem Patienten, verstellt bzw. ist hierzu eingerichtet bzw. vorgesehen, was vorliegend auch als Vorpositionieren der Roboterbasis (vor der Operation durch den Roboter) bezeichnet wird. Zusätzlich oder alternativ ist bzw. wird der Positioniervorrichtungsflansch während des Operationsbetriebs, insbesondere bei Stillstand des Endeffektors am oder im Patienten, verstellt bzw. ist hierzu eingerichtet bzw. vorgesehen, was vorliegend auch als Umpositionieren der Roboterbasis (während der Operation durch den Roboter) bezeichnet wird. Entsprechend betrifft die vorliegende Erfindung insbesondere ein Vor- und/oder Umpositionieren des Roboters.

In einer Ausführung ist bzw. wird die Roboterbasis starr bzw. unbeweglich mit dem Positioniervorrichtungsflansch verbindbar bzw. verbunden. In einer anderen Ausführung ist bzw. wird die Roboterbasis linear beweglich, insbesondere durch eine zu der Kinematik zusätzliche Linearachse bzw. ein weiteres Lineargelenk zusätzlich zu den Gelenken der Kinematik mit dem Positioniervorrichtungsflansch verbindbar bzw. verbunden. Hierdurch kann insbesondere ein Radius einer Kreisbahn, auf der der Positioniervorrichtungsflansch durch die Kinematik und das Orientierungsmittel geführt wird, variiert oder eine Gerade, entlang der der Positioniervorrichtungsflansch durch die Kinematik und das Orientierungsmittel geführt wird, parallel verschoben werden. Vorzugsweise ist diese zusätzliche Linearachse fixierbar und/oder parallel oder senkrecht zu einer ersten bzw. Basisdrehachse des mit dem Positioniervorrichtungsflansch verbundenen Roboters. Zusätzlich oder alternativ ist bzw. wird in einer Ausführung die Roboterbasis drehbar, insbesondere durch ein weiteres Drehgelenk zusätzlich zu den Gelenken der Kinematik mit dem Positioniervorrichtungsflansch verbindbar bzw. verbunden. Hierdurch kann insbesondere eine Orientierung der Roboterbasis relativ zu dem Positioniervorrichtungsflansch variiert werden. Vorzugsweise ist dieses zusätzliche Drehgelenk fixierbar und/oder seine Drehachse parallel oder senkrecht zu einer ersten bzw. Basisdrehachse des mit dem Positioniervorrichtungsflansch verbundenen Roboters.

Nach einem Aspekt der vorliegenden Erfindung weist die Positioniervorrichtung ein Orientierungsmittel auf, das den Positioniervorrichtungsflansch infolge bzw. in Abhängigkeit von einer, insbesondere manuellen oder aktuierten, Verstellung des Positioniervorrichtungsflanschs von der ersten in die zweite Position von einer ersten Orientierung, insbesondere Winkellage, in der ersten Position in eine zweite Orientierung, insbesondere Winkellage, in der zweiten Position umorientiert bzw. hierzu eingerichtet bzw. vorgesehen ist, die in einer Weiterbildung gegenüber der ersten Orientierung um wenigstens 75°, insbesondere wenigstens 85°, vorzugsweise mehr als 90° um eine Referenzachse, insbesondere eine Horizontale, verdreht ist.

Hierdurch kann in einer Ausführung auf einfache und/oder zuverlässige Weise, insbesondere auch durch medizinisches Personal ohne entsprechende kinematische Kenntnisse, der Roboter bzw. dessen Basis positioniert, insbesondere wahlweise wand- oder decken- bzw. bodenseitig angeordnet werden, insbesondere eine erste bzw. Basisdrehachse des Roboters wahlweise, wenigstens im Wesentlichen, horizontal oder vertikal.

Das Orientierungsmittel, das diese Umorientierung infolge, insbesondere bei bzw. während der Verstellung von der ersten in die zweite Position bewirkt, reduziert für den Anwender vorteilhaft die Anzahl der Freiheitsgrade, die er einstellen muss: er muss lediglich den Positioniervorrichtungsflansch bzw. die damit verbundene Roboterbasis, insbesondere translatorisch, in die erste oder zweite Position verstellen, insbesondere ziehen oder schieben, und das Orientierungsmittel bewirkt dabei zwangsläufig eine entsprechende Umorientierung, insbesondere Drehung, des Positioniervorrichtungsflanschs bzw. der damit verbundenen Roboterbasis von der ersten in die zweite Orientierung. Umgekehrt kann das Orientierungsmittel in einer Ausführung auch eine Verstellung von der ersten in die zweite Position infolge einer Umorientierung von der ersten in die zweite Orientierung bewirken. Durch den bevorzugten Winkelbereich von wenigstens 75°, insbesondere wenigstens 85°, vorzugsweise mehr als 90°, kann dabei in einer Ausführung zwischen einer wenigstens im Wesentlichen, horizontalen und vertikalen Orientierung gewechselt werden.

In einer Ausführung wird bzw. ist der Positioniervorrichtungsflansch durch die Kinematik in eine oder mehrere Zwischenpositionen zwischen der ersten und zweiten Position relativ zu der Positioniervorrichtungsbasis verstellt bzw. verstellbar, wobei das Orientierungsmittel den Flansches infolge einer Verstellung von der ersten oder zweiten Position in eine solche Zwischenposition in eine Zwischenorientierung in der Zwischenposition umorientiert bzw. hierzu eingerichtet bzw. vorgesehen ist, die in einer Weiterbildung gegenüber der ersten und/oder zweiten Orientierung um wenigstens 25° und/oder höchstens 60° um die Referenzachse verdreht ist.

Hierdurch kann in einer Ausführung der Roboter bzw. dessen Basis in gleicher Weise wahlweise auch in einer oder mehreren Zwischenlagen zwischen einer wand- und einer decken- bzw. bodenseitigen Lage angeordnet, insbesondere eine erste bzw. Basisdrehachse des Roboters wahlweise gegen die Horizontale und Vertikale gekippt werden bzw. sein.

In einer Ausführung ist der Positioniervorrichtungsflansch maximal zwischen der ersten und zweiten Position verstellbar, die somit einen maximalen Verstellweg des Positioniervorrichtungsflanschs definieren bzw. begrenzen. In einer alternativen Ausführung ist der Positioniervorrichtungsflansch hingegen über die erste und/oder zweite Position hinaus verstellbar, so dass die erste und zweite Position einen Mindest-Verstellweg des Positioniervorrichtungsflanschs definieren bzw. vorgeben.

In einer Ausführung ist die Kinematik bzw. der Positioniervorrichtungsflansch wenigstens in der ersten und/oder zweiten Position fixierbar, vorzugsweise auch in einer oder mehreren Zwischenpositionen, insbesondere in diskreten, vorgegebenen Zwischenpositionen oder stufenlos in beliebigen Zwischenpositionen zwischen der ersten und zweiten Position.

In einer Ausführung weist die Kinematik wenigstens ein Drehgelenk, insbesondere wenigstens drei, insbesondere seriell angeordnete, Drehgelenke, insbesondere mit parallelen Drehachsen auf. Zusätzlich oder alternativ zu einem oder mehreren Drehgelenken weist die Kinematik in einer Ausführung wenigstens ein Lineargelenk bzw. eine Linearachse auf.

Durch ein oder mehrere Drehgelenke, insbesondere in Kombination mit einer oder mehreren Linearachsen, kann in einer Ausführung ein vorteilhafter Arbeitsraum der Kinematik und/oder eine konstruktiv vorteilhafte Kinematik zur Verfügung gestellt werden.

In einer Ausführung weist die Kinematik drei seriell angeordnete bzw. durch, insbesondere starre, Glieder der Kinematik paarweise verbundene Drehgelenke mit parallelen Drehachsen auf. Wie nachfolgend näher erläutert, können hierdurch insbesondere zwei Parallelogramme bzw. eine Viergelenkführung mit den drei Drehgelenken der Kinematik und einem relativ zu der Positioniervorrichtungsbasis ortsfesten virtuellen Drehpunkt dargestellt werden, die den Positioniervorrichtungsflansch stets auf den virtuellen Drehpunkt ausrichtet.

In einer anderen Ausführung weist die Kinematik ein Drehgelenk und ein Lineargelenk bzw. eine Linearachse auf, die in einer Weiterbildung durch ein, insbesondere starres, Glied der Kinematik verbunden sind. Wie ebenfalls nachfolgend näher erläutert, kann hierdurch eine Galgenführung dargestellt werden, die den Positioniervorrichtungsflansch näherungsweise auf einer Geraden führt, jedoch gegenüber einer Viergelenkführung insbesondere konstruktiv vorteilhaft sein kann.

In einer Ausführung weist das Orientierungsmittel eine entsprechend, insbesondere software- bzw. programmtechnisch, eingerichtete Steuerung und durch diese kommandierte Aktuatoren auf bzw. ist hierdurch implementiert, die dazu vorgesehen bzw. eingerichtet ist, Gelenke, insbesondere Drehgelenke und/oder Linearachsen, der Kinematik durch entsprechend gesteuerte Aktuatoren, insbesondere Elektromotoren, derart zu bewegen, dass der Positioniervorrichtungsflansch infolge einer Verstellung des Positioniervorrichtungsflanschs von der ersten in die zweite Position von der ersten in die zweite Orientierung bzw. infolge einer Verstellung in eine Zwischenposition in die entsprechende Zwischenorientierung umorientiert wird. Dabei kann eine Verstellung des Positioniervorrichtungsflanschs in einer Ausführung manuell erfolgen und durch entsprechende Gelenksensoren der Kinematik erfasst und an die Steuerung übermittelt werden, die auf Basis der erfassten Verstellung die Aktuatoren steuert, um die entsprechende Umorientierung zu bewirken. Gleichermaßen kann eine Verstellung des Positioniervorrichtungsflanschs in einer Ausführung durch die Steuerung erfolgen, die dabei die Aktuatoren so steuert, dass dabei auch die entsprechende Umorientierung bewirkt wird.

Hierdurch kann in einer Ausführung ein besonders flexibles Orientierungsmittel zur Verfügung gestellt werden.

In einer anderen Ausführung ist das Orientierungsmittel mechanisch implementiert bzw. ausgebildet. Hierdurch kann in einer Ausführung ein besonders einfaches, zuverlässiges und/oder energieversorgungsunabhängiges Orientierungsmittel zur Verfügung gestellt werden, welches den Positioniervorrichtungsflansch insbesondere bei einer manuellen Verstellung in die zweite oder eine Zwischenposition zwnagslufig in die zweite bzw. Zwischenorientierung umorientiert.

Entsprechend weist in einer Ausführung das Orientierungsmittel eine oder mehrere mechanische Zwangskopplungen bzw. -führungen von je zwei Gliedern der Positioniervorrichtung auf, welche jeweils durch ein oder mehrere, insbesondere wenigstens zwei, Gelenke der Kinematik verbunden sind, insbesondere eine mechanische Zwangskopplung des Positioniervorrichtungsflansches mit einem Glied der Kinematik und/oder eine mechanische Zwangskopplung der Positioniervorrichtungsbasis mit einem (anderen) Glied der Kinematik oder eine mechanische Zwangskopplung der Positioniervorrichtungsbasis mit dem Positioniervorrichtungsflansch.

Eine mechanische Zwangskopplung bzw. -führung im Sinne der vorliegenden Erfindung reduziert mechanisch, insbesondere form- oder reibschlüssig, kinematische Freiheitsgrade der durch das bzw. die Gelenk(e) verbundenen Glieder um wenigstens eins, insbesondere auf Null, d.h. koppelt das eine Glied eineindeutig bzw. zwangsläufig an das mit ihm durch das bzw. die Gelenke verbundene andere Glied der Positioniervorrichtung, so dass eine Orientierung des einen Gliedes die Orientierung des anderen Gliedes zwangsläufig bestimmt.

Eine mechanische Zwangskopplung kann entsprechend in einer Ausführung insbesondere ein ein- oder mehrstufiges Getriebe, insbesondere ein Räder-, Zugmittel-, Schrauben-, Kurven-, Koppel-, insbesondere Schubstangen-, Kolbenstangen- oder Koppelstangen-, oder ein hydraulisches bzw. Fluidgetriebe, aufweisen, insbesondere sein. In einer Weiterbildung beträgt eine Übersetzung des Getriebes eins, so dass eine Orientierungsänderung des einen Gliedes identisch bzw. größengleich in eine Orientierungsänderung des anderen Gliedes umgesetzt wird.

In einer Ausführung sind bzw. werden eine oder mehrere mechanische Zwangskopplungen, insbesondere manuell und/oder aktuiert, insbesondere pneumatisch, hydraulisch, elektromotorisch und/oder -magnetisch, lösbar bzw. gelöst, beispielsweise durch Entkoppeln eines Getriebes oder dergleichen. Hierdurch kann in einer Ausführung vorteilhaft die Kinematik bei Nichtbedarf kompakt gefaltet werden.

In einer Ausführung sind die Positioniervorrichtungsbasis und der Positioniervorrichtungsflansch durch drei seriell angeordnete Drehgelenke mit parallelen Drehachsen verbunden, wobei durch das Orientierungsmittel eine, insbesondere mechanische, Viergelenk-, insbesondere Parallelogrammführung gebildet wird bzw. ist, die die drei seriell angeordneten Drehgelenke der Kinematik und einen relativ zu der Positioniervorrichtungsbasis ortsfesten virtuellen Drehpunkt mit parallelen Drehachsen aufweist. Durch eine solche Viergelenk-, insbesondere Parallelogrammführung, ist bzw. bleibt der Positioniervorrichtungsflansch bei einer translatorischen Verstellung auf einer Kreisbahn vorteilhaft stets auf den virtuellen Drehpunkt ausrichtet.

In einer Weiterbildung weist die Kinematik ein erstes Drehgelenk, welches die Positioniervorrichtungsbasis mit einem ersten Glied der Kinematik verbindet, ein zweites Drehgelenk, welches das erste mit einem zweiten Glied der Kinematik verbindet, und ein drittes Drehgelenk auf, welches das zweite Glied der Kinematik mit dem Positioniervorrichtungsflansch verbindet, und das Orientierungsmittel weist eine mechanische Zwangskopplung der Positioniervorrichtungsbasis mit diesem zweiten Glied der Kinematik und eine weitere Zwangskopplung des Positioniervorrichtungsflansches mit diesem ersten Glied der Kinematik auf.

In einer anderen Ausführung sind die Positioniervorrichtungsbasis und der Positioniervorrichtungsflansch durch ein Lineargelenk bzw. eine Linearachse und ein damit verbundenes Drehgelenk verbunden, wobei durch das Orientierungsmittel eine mechanische Galgen- bzw. Auslegerführung gebildet wird bzw. ist, die den Positioniervorrichtungsflansch bei einer translatorischen Verstellung auf einer Geraden näherungsweise auf einen virtuellen Drehpunkt ausrichtet bzw. einen positioniervorrichtungsflanschfesten Referenzpunkt innerhalb eines begrenzten Kreises hält.

In einer Weiterbildung weist die Kinematik ein Lineargelenk, welches die Positioniervorrichtungsbasis mit einem ersten Glied der Kinematik verbindet, und ein Drehgelenk auf, welches das erste Glied der Kinematik mit dem Positioniervorrichtungsflansch verbindet, und das Orientierungsmittel weist eine mechanische Zwangskopplung der Positioniervorrichtungsbasis mit dem Positioniervorrichtungsflansch auf. In einer Ausführung ist das Lineargelenk drehbar mit der Positioniervorrichtungsbasis verbunden. Hierdurch kann vorteilhafterweise eine Neigung der Geraden, längs der der Positioniervorrichtungsflansch verstellbar ist, relativ zu der Positioniervorrichtungsbasis variiert werden, insbesondere auch, um die Positioniervorrichtung in eine kompakte Transport- und/oder Lagerstellung zu bringen. In einer Ausführung weisen wenigstens ein oder mehrere, insbesondere alle, Gelenke der Kinematik eine Verriegelung zum Blockieren bzw. Fixieren des jeweiligen Gelenks auf. Durch ein wahlweises Blockieren von einem oder mehreren Gelenken kann die Anzahl der Freiheitsgrade der Kinematik reduziert werden, durch ein Blockieren aller Gelenke der Kinematik insbesondere auf Null bzw. die Kinematik und damit der Positioniervorrichtungsflansch relativ zu der Positioniervorrichtungsbasis fixiert werden, insbesondere in der ersten Stellung, der zweiten Stellung oder wenigstens einer Zwischenstellung, vorzugsweise in einer beliebigen Zwischenstellung zwischen der ersten und zweiten Stellung, die in einer Ausführung durch die maximalen Auslenkungen bzw. Verstellwege der Kinematik gegeben sind.

Nach einem Aspekt der vorliegenden Erfindung reduziert das Orientierungsmittel die durch die Freiheitsgrade der Gelenke der Kinematik gegebene Anzahl der Freiheitsgrade der Positioniervorrichtung zwischen Positioniervorrichtungsbasis und - flansch um wenigstens eins, vorzugsweise auf eins.

Entsprechend würde bereits das Blockieren eines Gelenkes diesen verbliebenen Freiheitsgrad eliminieren und den Positioniervorrichtungsflansch in der entsprechenden Position und Orientierung relativ zu der Positioniervorrichtungsbasis fixieren. Durch das zusätzliche Blockieren weiterer, insbesondere aller Gelenke der Kinematik können in einer Ausführung vorteilhaft das Orientierungsmittel, insbesondere eine oder mehrere mechanische Zwangskopplungen, entlastet werden. Zusätzlich oder alternativ kann ein solches Blockieren vorteilhaft den Positioniervorrichtungsflansch redundant fixieren. Insbesondere können durch eine solche redundante Fixierung das Orientierungsmittel, insbesondere eine oder mehrere mechanische Zwangskopplungen, insbesondere Getriebe, größere Toleranzen und/oder Nachgiebigkeiten aufweisen, da der Roboter diese, insbesondere nach einer Kalibrierung, in der durch die blockierten Gelenke präzise fixierten Lage seiner Basis kompensieren kann.

In einer Ausführung werden bzw. sind die Gelenke mechanisch blockiert bzw. blockierbar, insbesondere form- oder reibschlüssig, etwa durch Schließen von Riegeln, Feststellbremsen oder dergleichen.

In einer Ausführung werden bzw. sind die Gelenke manuell blockiert bzw. blockierbar. Dies erleichtert insbesondere die direkte Handhabung der Positioniervorrichtung. Zusätzlich oder alternativ werden bzw. sind die Gelenke in einer Ausführung aktuiert blockiert bzw. blockierbar, beispielsweise durch elektromagnetisches Schließen oder Lüften von Bremsen, Riegeln oder dergleichen. Dies erleichtert insbesondere das Blockieren schwer zugänglicher Gelenke.

In einer Ausführung werden bzw. sind ein oder mehrere Gelenke zusammen mit wenigstens einem anderen Gelenk der Kinematik blockiert bzw. blockierbar, insbesondere alle Gelenke der Kinematik zusammen blockiert bzw. blockierbar. Hierdurch kann eine gewünschte Stellung unmittelbar redundant fixiert und das Orientierungsmittel entlastet werden. Alternativ oder zusätzlich, insbesondere in einem anderen Betriebsmodus, werden bzw. sind ein oder mehrere Gelenke in einer Ausführung unabhängig von wenigstens einem anderen Gelenk der Kinematik blockiert bzw. blockierbar. Hierdurch kann in einer Ausführung einerseits bereits, wie vorstehend erläutert, zusammenwirkend mit dem Orientierungsmittel die Lage des Positioniervorrichtungsflanschs fixiert werden. Auf der anderen Seite können das bzw. die anderen Gelenke vorteilhaft weiter bewegt werden, wenn das Orientierungsmittel wirkungslos ist, beispielsweise durch Lösen einer oder mehreren mechanischer Zwangskopplungen oder dergleichen.

In einer Ausführung weist die Positioniervorrichtung wenigstens ein, insbesondere mechanisches und/oder pneumatisches, Spannmittel auf, das zwei durch wenigstens ein Gelenk der Kinematik miteinander verbundene Glieder der Positioniervorrichtung miteinander fesselt und durch eine Bewegung der Positioniervorrichtung in eine Lage, in der sie eine niedrigere Lageenergie aufweist, insbesondere durch ein Verstellen des Positioniervorrichtungsflanschs von einer angehobenen Stellung in eine abgesenkte Stellung, stärker gespannt wird. Hierdurch wird vorteilhaft ein, wenigstens teilweiser, Gewichtsausgleich zur Verfügung gestellt, der ein Verstellen des Positioniervorrichtungsflanschs erleichtert, indem Energie zwischen Lageenergie und im Spannmittel gespeicherter Energie transferiert wird. Mit anderen Worten wird bzw. ist das Spannmittel in einer Ausführung in derjenigen von der ersten und zweiten Stellung des Positioniervorrichtungsflansches, in der die Positioniervorrichtung die niedrigere Lageenergie aufweist, stärker gespannt als in der anderen von der ersten und zweiten Stellung des Positioniervorrichtungsflansches, in der die Positioniervorrichtung die demgegenüber größere Lageenergie aufweist.

In einer Ausführung weist die Positioniervorrichtung wenigstens zwei Glieder auf, die durch wenigstens ein Gelenk der Kinematik miteinander verbunden und in eine Lagerstellung zusammenfaltbar sind, in der diese beiden Glieder, wenigstens im Wesentlichen, gegensinnig parallel zueinander sind. Hierdurch kann die Positioniervorrichtung bei Nichtgebrauch vorteilhaft kompakt verstaut und gelagert werden.

In einer Ausführung weist die Positioniervorrichtung einen Träger auf, an dem die Positioniervorrichtungsbasis ein- oder mehrachsig linear beweglich und/oder drehbar gelagert ist. Hierdurch kann in einer Ausführung die Positioniervorrichtungsbasis ihrerseits gegenüber dem Träger vorpositioniert werden. Dies ermöglicht es vorteilhaft, die Positioniervorrichtung an verschiedene Operationssäle oder -gebiete mit entsprechend unterschiedlichen Patientenlagerungen und -zugängen besser anzupassen und insbesondere zusätzlich zu dem vorstehend erläutertem Wechsel zwischen einer wand- und einer decken- oder bodenseitigen bzw. horizontalen oder vertikal hängenden oder stehenden Ausrichtung der ersten bzw. Basisachse des Roboters Interferenzen zwischen mehreren gemeinsam agierenden Robotern zu vermeiden.

Der Träger ist in einer Ausführung stationär, insbesondere an einer Wand, Decke oder einem Boden eines Operationssaals dauerhaft oder lösbar befestigt. In einer anderen Ausführung ist der Träger, insbesondere manuell, beweglich bzw. mobil, insbesondere durch die hier beschriebene Luftkammeranordnung, ein entsprechendes, insbesondere antriebsloses Fahrwerk oder dergleichen. Gleichermaßen kann der Träger auch automobil sein, etwa durch ein entsprechendes, insbesondere motorisch angetriebenes, Fahrwerk oder dergleichen.

In einer Ausführung ist die Positioniervorrichtungsbasis in einer Betriebsstellung des Trägers um eine vertikale und/oder horizontale Achse drehbar. Dies ermöglicht insbesondere eine vorteilhafte Ausrichtung relativ zur Umgebung. Zusätzlich oder alternativ ist die Positioniervorrichtungsbasis um eine Achse drehbar, die senkrecht oder parallel zu einer Achse des Gelenks der Kinematik ist, mit dem die Positioniervorrichtungsbasis verbunden ist. Dies ermöglicht insbesondere eine vorteilhafte Ausrichtung von Robotern untereinander.

Zusätzlich oder alternativ ist die Positioniervorrichtungsbasis in einer Betriebsstellung des Trägers vertikal und/oder horizontal linear beweglich. Dies ermöglicht insbesondere eine vorteilhafte Ausrichtung relativ zur Umgebung. Zusätzlich oder alternativ ist die Positioniervorrichtungsbasis in bzw. entlang einer Achse linear beweglich, die senkrecht oder parallel zu einer Achse des Gelenks der Kinematik ist, mit dem die Positioniervorrichtungsbasis verbunden ist. Dies ermöglicht insbesondere eine vorteilhafte Ausrichtung von Robotern untereinander.

In einer Ausführung ist die Positioniervorrichtungsbasis an dem Träger in einer oder mehreren Positionen und/oder Orientierungen fixierbar, so dass die an dem Positioniervorrichtungsflansch befestigte Roboterbasis über die Positioniervorrichtung und den Träger umgebungsfestgelegt werden kann.

Weist eine Roboteranordnung in einer Ausführung wenigstens zwei der hier beschriebenen Positioniervorrichtungen auf, an deren Positioniervorrichtungsflanschen jeweils einer der hier beschriebenen Roboter befestigtbar bzw. befestigt sind bzw. werden, so können in einer Weiterbildung die Positioniervorrichtungsbasen von wenigstens zwei der Positioniervorrichtungen gemeinsam in der hier beschriebenen Weise ein- oder mehrachsig linear beweglich und/oder drehbar an dem Träger gelagert sein. Gleichermaßen können in einer anderen Weiterbildung die Positioniervorrichtungsbasen von wenigstens zwei der Positioniervorrichtungen unabhängig voneinander in der hier beschriebenen Weise ein- oder mehrachsig linear beweglich und/oder drehbar an demselben Träger oder an voneinander unabhängigen Trägern gelagert sein.

In einer Ausführung weist der Träger ein Gehäuse auf, in das die Positioniervorrichtungsbasis, die, insbesondere hierzu gefaltete, Kinematik, und/oder der Positioniervorrichtungsflansch vollständig oder teilweise einfahrbar ist. Hierdurch wird in einer Ausführung eine kompakte Aufbewahrungs- und/oder Transportlage zur Verfügung gestellt, in der die in das Gehäuse eingefahrenen Bereiche der Positioniervorrichtung und die Umwelt, insbesondere Bedienpersonal, gegeneinander geschützt sind.

Nach einem Aspekt der vorliegenden Erfindung weist eine Roboteranordnung eine hier beschriebene Positioniervorrichtung sowie einen hier beschriebenen Roboter auf, der bzw. dessen Basis, insbesondere lösbar, an dem Positioniervorrichtungsflansch befestigbar bzw. befestigt ist bzw. wird.

Nach einem weiteren Aspekt der vorliegenden Erfindung wird zum Positionieren eines hier beschriebenen Roboters einer Roboteranordnung mit einer Positioniervorrichtung, die eine Basis und einen Flansch aufweist, an dem der Roboter befestigt ist bzw. wird, wobei der Flansch durch eine Kinematik mit der Basis verbunden ist, durch die der Flansch von einer ersten Position relativ zu der Basis in eine hiervon beabstandete zweite Position relativ zu der Basis verstellbar und/oder von einer ersten Orientierung relativ zu einer Referenzachse in eine gegenüber der ersten Orientierung verdrehte zweite Orientierung relativ zu der Referenzachse umorientierbar ist, insbesondere einer Positioniervorrichtung nach einem anderen der hier beschriebenen Aspekte, der Roboter zum, insbesondere manuellen, Verstellen und/oder Umorientieren des Positioniervorrichtungsflanschs in eine vorgegebene Positionierungspose gesteuert, insbesondere durch entsprechende Aktuierung seiner Gelenkantriebe.

Hierdurch kann vorteilhaft die Vor- und/oder Umpositionierung des Roboters erleichtert werden.

Die vorgegebene Positionierungspose ist in einer Ausführung eine Pose, in der der Roboter eine vorteilhafte Manipulierbar- bzw. Beweglichkeit aufweist, insbesondere ein oder mehrere Gelenke des Roboters jeweils gegensinnig, insbesondere wenigstens im Wesentlichen betragsgleich, beweglich sind. So kann beispielsweise ein Drehgelenk, welches einen Winkelbereich von 180° aufweist, in der vorgegebenen Positionierungspose eine Winkelstellung von etwa 90° aufweisen. Zusätzlich oder alternativ, insbesondere bei Gelenken mit unendlicher Beweglichkeit, kann eine vorgegebene Positionierungspose , in der der Roboter eine vorteilhafte Manipulierbar- bzw. Beweglichkeit aufweist, beispielsweise durch einen großen, insbesondere maximalen Abstand zu einer singulären Pose des Roboters oder dadurch gekennzeichnet sein, dass der Roboter in der Pose bevorzugte, insbesondere statistisch häufige, Endeffektorbewegungen durch ausgesuchte Gelenke oder Gelenkkombinationen ausführen kann.

In einer Ausführung wird die vorgegebene Positionierungspose, beispielsweise auf Basis einer durchzuführenden Operation und/oder einer Lage des Positioniervorrichtungsflansches relativ zur Positioniervorrichtungsbasis und/oder einer Lage der Positioniervorrichtungsbasis relativ zum Träger, aus mehreren vorgegebenen Posen ausgewählt. Hierdurch kann der Benutzer den Roboter in einer für den jeweiligen Anwendungsfall optimalen Pose vorpositionieren.

Zusätzlich oder alternativ zu einem Steuern des Roboters in eine vorgegebene Positionierungspose zum, insbesondere manuellen, Verstellen und/oder Umorientieren des Positioniervorrichtungsflanschs wird nach einem weiteren Aspekt der vorliegenden Erfindung nach einem Fixieren der Positioniervorrichtung, insbesondere einem Fixieren des Positioniervorrichtungsflanschs in einer Lage relativ zu der Positioniervorrichtungsbasis und/oder der Positioniervorrichtungsbasis relativ zu der Umgebung, ein, insbesondere positioniervorrichtungsbasis-, träger- oder umgebungsfeste Kalibriermittel mit einem roboterfesten Referenzpunkt, insbesondere einem ausgezeichneten Punkt des Roboterflanschs oder -endeffektors, in einer oder mehreren voneinander verschiedenen Posen des Roboters angefahren und die zugehörigen Gelenkkoordinaten des Roboters ausgewertet. Auf diese Weise kann ein Bezugssystem einer Steuerung des Roboters kalibriert und so eine Vor- bzw. Umpositionierung seiner Roboterbasis kompensiert werden.

Wie bereits ausdrücklich betont, können der bzw. die hier beschriebenen Roboter insbesondere Chirurgieroboter mit einem chirurgischen Endeffektor sein. In den Ausführungsbeispielen werden daher Chirurgieroboter verwendet, wofür die vorliegende Erfindung besonders vorteilhaft ist. Gleichermaßen können der bzw. die hier beschriebenen Manipulatoren, insbesondere Roboter, auch Industrie- und/oder Servicemanipulatoren, insbesondere -roboter sein, für die die vorliegende Erfindung ebenfalls vorteilhaft ist.

Weitere Vorteile und Merkmale ergeben sich aus den Unteransprüchen und Ausführungsbeispielen. Hierzu zeigen, teilweise schematisiert:
- Fig. 1:: eine Positioniervorrichtung nach einer Ausführung der vorliegenden Erfindung mit einem Orientierungsmittel;
- Fig. 2:: die Positioniervorrichtung der Fig. 1, wobei das Orientierungsmittel zwei Koppelstangen aufweist;
- Fig. 3:: die Positioniervorrichtung der Fig. 1, wobei das Orientierungsmittel zwei Zugmittelgetriebe aufweist;
- Fig. 4A - Fig. 4E:: einen Lagervorgang einer Chirurgieroboteranordnung nach einer Ausführung der vorliegenden Erfindung;
- Fig. 5:: eine Positioniervorrichtung nach einer weiteren Ausführung der vorliegenden Erfindung;
- Fig. 6:: eine Positioniervorrichtung nach einer weiteren Ausführung der vorliegenden Erfindung;
- Fig. 7:: eine Chirurgieroboteranordnung nach einer Ausführung der vorliegenden Erfindung in perspektivischer Darstellung;
- Fig. 8:: eine Chirurgieroboteranordnung nach einer weiteren Ausführung der vorliegenden Erfindung in perspektivischer Darstellung;
- Fig. 9:: eine Chirurgieroboteranordnung nach einer weiteren Ausführung der vorliegenden Erfindung in perspektivischer Darstellung;
- Fig. 10:: eine Chirurgieroboteranordnung nach einer weiteren Ausführung der vorliegenden Erfindung in perspektivischer Darstellung;
- Fig. 11:: eine Manipulatoranordnung mit einem Trägersystem nach einer Ausführung der vorliegenden Erfindung in einer Seitansicht;
- Fig. 12:: einen Teil des Trägersystems der Fig. 11 in einem Schnitt;
- Fig. 13:: einen Teil eines Trägersystems nach einer weiteren Ausführung der vorliegenden Erfindung in Fig. 12 entsprechender Ansicht;
- Fig. 14:: einen Teil eines Trägersystems nach einer weiteren Ausführung der vorliegenden Erfindung in Fig. 12, 13 entsprechender Ansicht; und
- Fig. 15:: ein Verfahren zum Bewegen und/oder Fixieren des Trägersystems der Fig. 13 nach einer Ausführung der vorliegenden Erfindung.

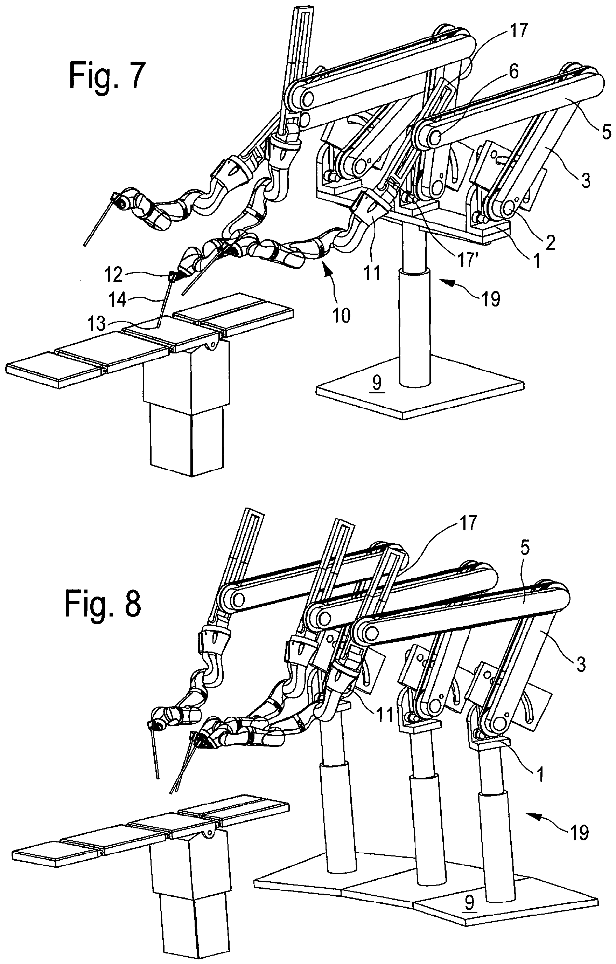

Fig. 7 zeigt eine Chirurgieroboteranordnung nach einer Ausführung der vorliegenden Erfindung in perspektivischer Darstellung. Diese weist einen Träger 9 auf, an dem drei Positioniervorrichtungen gemeinsam einachsig linear beweglich und drehbar gelagert sind, an denen wiederum jeweils ein Chirurgieroboter befestigt ist. Die Positioniervorrichtungen und Chirurgieroboter sind jeweils baugleich, so dass nachfolgend stellvertretend nur die bzw. den in Fig. 7 vordere(n) näher erläutert wird.

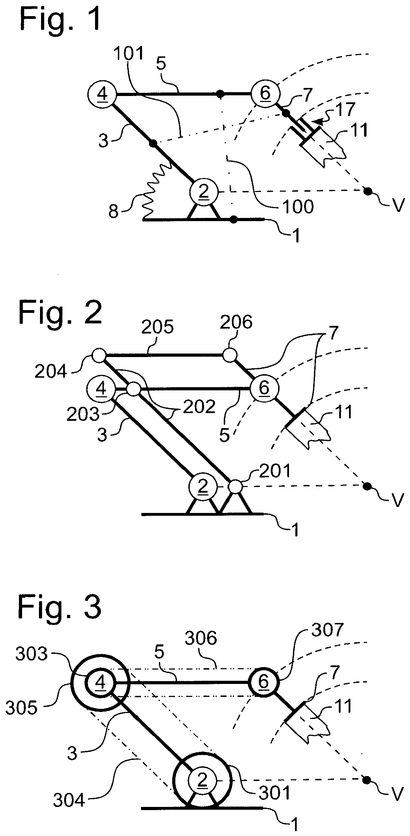

Diese Positioniervorrichtung weist eine Positioniervorrichtungsbasis 1 und einen Positioniervorrichtungsflansch 7 (vgl. Fig. 1) auf, an dem der siebenachsige Chirurgieroboter 10 befestigt ist.

Der Chirurgieroboter 10 ist für die minimalinvasive Chirurgie vorgesehen und weist hierzu einen chirurgischen Endeffektor 13 auf, der über einen Instrumentenschaft 14 an einem Chirurgieroboterflansch 12 des Chirurgieroboters befestigt ist, der seinerseits durch sieben Drehgelenke mit einer Chirurgieroboterbasis 11 verbunden ist, die an dem Positioniervorrichtungsflansch 7 befestigt ist (vgl. Fig. 1).

Wie insbesondere in Fig. 1 erkennbar, in der eine Positioniervorrichtung schematisiert dargestellt ist, welche bis auf nachfolgend erläuterte Unterschiede der Positioniervorrichtung der Fig. 7 entspricht, ist der Positioniervorrichtungsflansch 7 durch eine Kinematik, die drei Drehgelenke 2, 4, 6 aufweist, mit der Positioniervorrichtungsbasis 1 verbunden.

Durch diese Kinematik ist der Positioniervorrichtungsflansch translatorisch von einer kartesischen ersten Position relativ zu der Positioniervorrichtungsbasis auf einer in Fig. 1 gestrichelt angedeuteten Kreisbahn in eine hiervon beabstandete zweite Position verstellbar. Die Gelenke der Kinematik sind durch starre Glieder 3, 5 miteinander verbunden

In der Ausführung der Fig. 1, 7 ist die Chirurgieroboterbasis 11 durch ein zu den Drehgelenken 2, 4, 6 der Kinematik zusätzliches, fixierbares Lineargelenk 17 linear beweglich mit dem Positioniervorrichtungsflansch 7 verbunden. Dieses ist in den Ausführungen der Fig. 2, 3 und 5 zur besseren Übersichtlichkeit nicht dargestellt.

In der Ausführung der Fig. 7 ist die Chirurgieroboterbasis 11 darüberhinaus auch noch durch ein zu den Gelenken 2, 4, 6 der Kinematik zusätzliches, fixierbares weiteres Drehgelenk 17'drehbar an dem Positioniervorrichtungsflansch 7 bzw. dem Abtrieb des Lineargelenks 17 befestigt. Die Drehachse dieses weiteren Drehgelenks 17' ist senkrecht zu einer ersten bzw. Basisdrehachse des Chirurgieroboters 10.

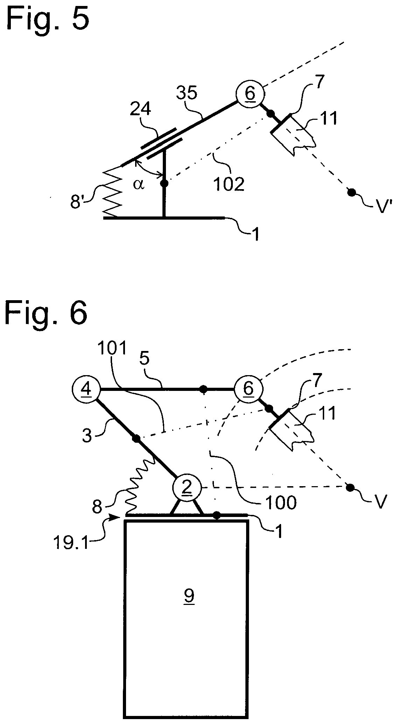

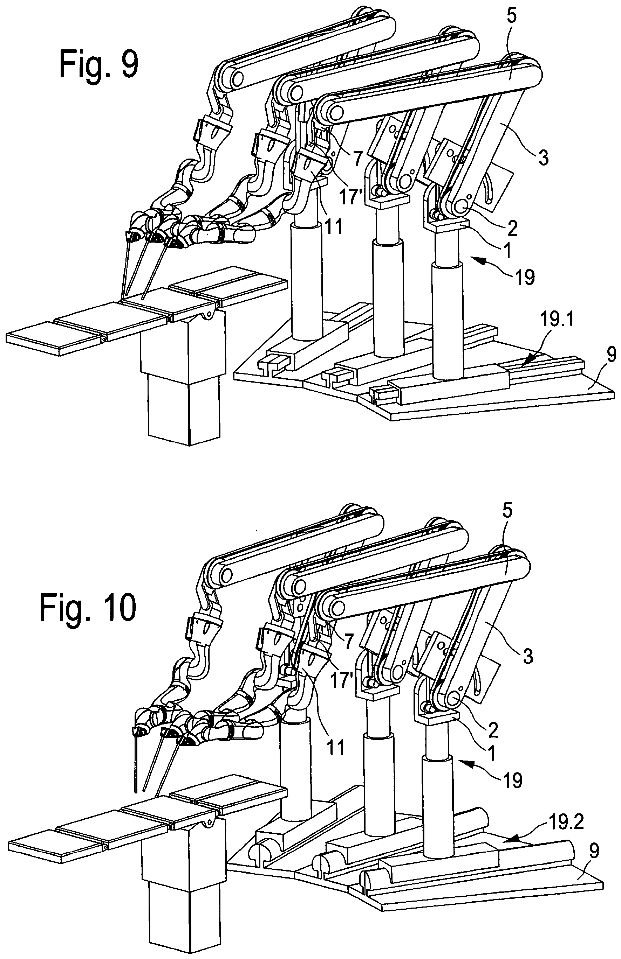

In der Ausführung der Fig. 6, die im Übrigen mit der Ausführung der Fig. 1 übereinstimmt, ist hingegen die Chirurgieroboterbasis 11 starr mit dem Positioniervorrichtungsflansch 7 verbunden. Dafür ist die Positioniervorrichtungsbasis 1 durch eine Linearachse 19.1, die nachfolgend mit Bezug auf Fig. 9 näher erläutert wird, horizontal beweglich an einem Träger 9 befestigt.

Wie diese Ausführungen zeigen, kann die Chirurgieroboterbasis 11starr (vgl. Fig. 6), linear beweglich (vgl. Fig. 1) und/oder drehbar (vgl. Fig. 7) mit dem Positioniervorrichtungsflansch 7 verbunden sein.

Wie nun insbesondere mit Bezug auf Fig. 1 bis 3 näher erläutert, weist die Positioniervorrichtung ein Orientierungsmittel auf, das den Positioniervorrichtungsflansch 7 infolge einer Verstellung des Positioniervorrichtungsflanschs von der ersten in die zweite Position von einer ersten Orientierung in der ersten Position in eine zweite Orientierung in der zweiten Position umorientiert, die gegenüber der ersten Orientierung um 90° um eine Horizontale verdreht ist.

Hierdurch kann in einer Ausführung auf einfache und zuverlässige Weise auch durch medizinisches Personal ohne entsprechende kinematische Kenntnisse der Chirurgieroboter 10 bzw. dessen Basis 11 wahlweise wand- oder deckenseitig angeordnet werden. Dabei reduziert das Orientierungsmittel die Anzahl der Freiheitsgrade, die der Anwender einstellen muss, so dass dieser lediglich den Positioniervorrichtungsflansch 7 bzw. die damit verbundene Chirurgieroboterbasis 11 in die erste oder zweite Position verstellen, insbesondere ziehen oder schieben, muss, während das Orientierungsmittel dabei zwangsläufig eine entsprechende Umorientierung des Positioniervorrichtungsflanschs 7 bzw. der damit verbundenen Chirurgieroboterbasis 11 von der ersten in die zweite Orientierung bewirkt. Dies ist in Fig. 1 durch einen virtuellen Drehpunkt V angedeutet, auf den der Positioniervorrichtungsflanschs 7 bei einer Verstellung auf der Kreisbahn ausgerichtet bleibt.

In den hier dargestellten Ausführungen ist das Orientierungsmittel jeweils mechanisch implementiert bzw. ausgebildet, wobei die Gelenke 2, 4, 6 passiv bzw. aktuatorlos sind. In einer nicht dargestellten Abwandlung kann es gleichermaßen durch eine Steuerung und durch diese kommandierte Aktuatoren implementiert sein, die diese Gelenke, welche in der Abwandlung dann aktuiert sind, durch die hierzu entsprechend gesteuerten Aktuatoren, insbesondere Elektromotoren, so zu bewegen wie dies nachfolgend für das mechanisch ausgebildete Orientierungsmittel beschrieben ist.

In der Ausführung der Fig. 1 und entsprechend auf der Fig. 6 und 7 weist das Orientierungsmittel eine mechanische Zwangskopplung 100 der Positioniervorrichtungsbasis 1 mit dem Glied 5 der Kinematik, die in Fig. 1 doppel-strichpunktiert schematisiert ist, und eine weitere mechanische Zwangskopplung 101 des Positioniervorrichtungsflansches 7 mit dem Glied 3 der Kinematik auf, die in Fig. 1 strichpunktiert schematisiert ist.

Diese mechanischen Zwangskopplungen können insbesondere ein ein- oder mehrstufiges Räder-, Zugmittel-, Schrauben-, Kurven-, Koppel-, insbesondere Schubstangen-, Kolbenstangen- oder Koppelstangen-, oder hydraulisches bzw. Fluidgetriebe, aufweisen.

Zur Verdeutlichung zeigt Fig. 2 exemplarisch eine Ausführung der Positioniervorrichtung der Fig. 1, in der die dort nur schematisierten mechanischen Zwangskopplungen 100, 101 durch Koppelstangengetriebe ausgebildet sind.

Diese weisen ein positioniervorrichtungsbasisfestes Drehgelenk 201 auf, das eine erste Koppelstange 202 drehbar lagert, die ihrerseits in einem Drehgelenk 203 an dem Glied 5 der Kinematik drehbar gelagert ist, so dass die Drehgelenke 2, 4, 201 und 203 mit den Gliedern 3 und 5 sowie der ersten Koppelstange 202 und der Positioniervorrichtungsbasis 1 eine Parallelogrammführung bilden. Die erste Koppelstange 202 ist in einem Drehgelenk 204 drehbar mit einer zweiten Koppelstange 205 verbunden, die ihrerseits in einem Drehgelenk 206 an dem Positioniervorrichtungsflansch 7 drehbar gelagert ist, so dass die Drehgelenke 6, 203, 204 und 206 mit dem Glieder 5, der ersten Koppelstange 202, der zweiten Koppelstange 205 und dem Positioniervorrichtungsflansch 7 eine weitere Parallelogrammführung bilden.

Fig. 3 zeigt zur Verdeutlichung exemplarisch eine andere Ausführung der Positioniervorrichtung der Fig. 1, in der die dort nur schematisierten mechanischen Zwangskopplungen 100, 101 durch Zugmittelgetriebe ausgebildet sind.

Diese weisen eine positioniervorrichtungsbasisfeste erste Synchronscheibe 301, deren Achse mit der Achse des Gelenks 2 fluchtet, eine starr mit dem Glied 5 verbundene zweite Synchronscheibe 305 , deren Achse mit der Achse des Gelenks 4 fluchtet und die über ein erstes Zugmittel 304 mit der erste Synchronscheibe 301 gekoppelt ist, eine starr mit dem Glied 3 verbundene dritte Synchronscheibe 303 , deren Achse ebenfalls mit der Achse des Gelenks 4 fluchtet, und eine starr mit dem Positioniervorrichtungsflansch 7 verbundene vierte Synchronscheibe 307 auf, deren Achse mit der Achse des Gelenks 6 fluchtet und die über ein zweites Zugmittel 306 mit der dritten Synchronscheibe 303 gekoppelt ist.

Man erkennt anhand der beiden exemplarischen Ausgestaltungen der mechanischen Zwangsführungen 100, 101 durch das Koppelstangen- bzw. Zugmittelgetriebe, dass hierdurch jeweils eine Umorientierung des Positioniervorrichtungsflansches 7 infolge einer Verstellung des Positioniervorrichtungsflansches 7 auf der gestrichelt angedeuteten Kreisbahn bewirkt wird, die den Positioniervorrichtungsflansch 7 stets auf den virtuellen Drehpunkt V ausrichtet. Dabei ist dem Fachmann klar, dass die mechanischen Zwangsführungen 100, 101 anstelle der gezeigten Ausgestaltungen auch anders realisiert sein können. Insbesondere können beispielsweise auch andere Koppelstangen- und Zugmittelgetriebe vorgesehen sein, beispielsweise das Zugmittelgetriebe 303, 306, 307 zur Kopplung des Gliedes 3 und des Positioniervorrichtungsflansches 7 und das Koppelstangengetriebe 2, 3, 4, 5, 203, 202, 201 und 1 zur Kopplung des Gliedes 5 und der Positioniervorrichtungsbasis 1, wobei dann die Elemente 203, 205 und 206 entfallen.

In der Ausführung der Fig. 1 und entsprechend der Fig. 2, 3, 6 und 7 sind die Positioniervorrichtungsbasis 1 und der Positioniervorrichtungsflansch 7 durch drei seriell angeordnete Drehgelenke 2, 4, 6 mit parallelen Drehachsen verbunden, wobei durch das Orientierungsmittel eine mechanische Parallelogrammführung gebildet ist, die die drei seriell angeordneten Drehgelenke der Kinematik und den relativ zu der Positioniervorrichtungsbasis 1 ortsfesten virtuellen Drehpunkt V mit parallelen Drehachsen aufweist. Entsprechend weist die Kinematik mit dem Drehgelenk 2 ein erstes Drehgelenk, welches die Positioniervorrichtungsbasis 1 mit dem (ersten) Glied 3 der Kinematik verbindet, mit dem Drehgelenk 4 ein zweites Drehgelenk, welches das (erste) Glied 3 mit dem (zweiten) Glied 5 der Kinematik verbindet, und mit dem Drehgelenk 6 ein drittes Drehgelenk auf, welches das (zweite) Glied 5 der Kinematik mit dem Positioniervorrichtungsflansch 7 verbindet, auf, das Orientierungsmittel weist eine mechanische Zwangskopplung 100 der Positioniervorrichtungsbasis 1 mit dem (zweiten) Glied 5 der Kinematik und eine weitere Zwangskopplung 101 des Positioniervorrichtungsflansches 7 mit dem (ersten) Glied 3 der Kinematik auf.

Fig. 5 zeigt in Fig. 1 entsprechender Weise eine Positioniervorrichtung nach einer weiteren Ausführung der vorliegenden Erfindung, die alternativ zu der mit Bezug auf Fig. 1-3 und 6 erläuterten Positioniervorrichtung verwendet werden kann, insbesondere in der Chirurgieroboteranordnung der Fig. 7. In dieser Ausführung sind die Positioniervorrichtungsbasis 1 und der Positioniervorrichtungsflansch 7 durch ein Lineargelenk bzw. eine Linearachse 24 und ein damit verbundenes Drehgelenk 6 verbunden, wobei durch das Orientierungsmittel eine mechanische Galgenführung gebildet ist, die den Positioniervorrichtungsflansch bei einer translatorischen Verstellung auf einer in Fig. 5 gestrichelten Geraden näherungsweise auf einen virtuellen Drehpunkt V' ausrichtet.

Hierzu weist die Kinematik das Lineargelenk 24, welches die Positioniervorrichtungsbasis 1 mit einem ersten Glied 35 der Kinematik verbindet, und ein Drehgelenk 6 auf, welches das erste Glied 35 der Kinematik mit dem Positioniervorrichtungsflansch 7 verbindet, wobei das Orientierungsmittel eine mechanische Zwangskopplung 102 der Positioniervorrichtungsbasis 1 mit dem Positioniervorrichtungsflansch 7 aufweist, die in Fig. 5 in Fig. 1 entsprechender Weise doppel-strichpunktiert schematisiert ist. In einer Abwandlung (nicht dargestellt) ist das Lineargelenk 24 drehbar mit der Positioniervorrichtungsbasis 1 verbunden. Hierdurch kann vorteilhafterweise eine Neigung bzw. ein Winkel α der Geraden, längs der der Positioniervorrichtungsflansch 7 verstellbar.ist, relativ zu der 1 Positioniervorrichtungsbasis variiert werden.

In den hier erläuterten Ausführungen weisen die unaktuierten Gelenke 2, 4, 6 bzw. 24 der Kinematik jeweils eine Verriegelung zum form- oder reibschlüssigen Blockieren bzw. Fixieren des jeweiligen Gelenks auf, etwa durch Schließen von Riegeln, Feststellbremsen oder dergleichen (nicht dargestellt).

In den hier erläuterten Ausführungen weisen die Positioniervorrichtungen jeweils ein, in den Fig. 1, 5 und 6 durch eine mechanische Feder 8 bzw. 8' angedeutetes, Spannmittel auf, das die zwei durch das Gelenk 2 der Kinematik miteinander verbundene Glieder 1, 3 bzw. die zwei durch das Gelenk 24 der Kinematik miteinander verbundene Glieder 1, 35 miteinander fesselt und durch eine Bewegung der Positioniervorrichtung in eine Lage, in der sie eine niedrigere Lageenergie aufweist, stärker gespannt wird. Hierdurch wird vorteilhaft ein, wenigstens teilweiser, Gewichtsausgleich zur Verfügung gestellt, der ein Verstellen des Positioniervorrichtungsflanschs erleichtert. In den übrigen Figuren sind die dort vorzugsweise ebenfalls vorhandenen Spannmittel zur besseren Übersichtlichkeit nicht dargestellt.

In der Ausführung der Fig. 7 weist die Positioniervorrichtung, wie einleitend erläutert, einen Träger 9 auf, an dem die Positioniervorrichtungsbasen 1 der drei Positioniervorrichtungen gemeinsam einachsig linear beweglich und drehbar gelagert ist. In der Ausführung der Fig. 7 ist der Träger 9 hierzu durch eine Hubsäule 19 mit einem Grundgerüst verbunden, an dem die Positioniervorrichtungsbasen 1 befestigt sind, wobei die Hubsäule 19 um ihre Längsachse drehbar ist.

Der Träger 9 ist in der Ausführung der Fig. 7 stationär. In einer Abwandlung ist der Träger mobil, insbesondere manuell beweglich.

In der Ausführung der Fig. 7 sind die Positioniervorrichtungsbasen 7 (bei einem portablen Träger in dessen Betriebsstellung) durch die Hubsäule 19 um eine vertikale Achse drehbar, welche senkrecht zu der Achse des Gelenks 2 der Kinematik ist, mit dem die Positioniervorrichtungsbasis 1 verbunden ist. Zusätzlich sind die Positioniervorrichtungsbasen 7 durch die Hubsäule 19 vertikal entlang einer Achse linear beweglich, welche senkrecht zu der Achse des Gelenks 2 der Kinematik ist, mit dem die Positioniervorrichtungsbasis 1 verbunden ist. Die Hubsäule ist arretier- bzw. fixierbar (nicht dargestellt).

Wie vorstehend ausgeführt, weist die Chirurgieroboteranordnung der Fig. 7 drei der insbesondere mit Bezug auf Fig. 1-3, 5 und 6 beschriebenen Positioniervorrichtungen auf, deren Positioniervorrichtungsbasen gemeinsam linear beweglich und/oder drehbar an dem Träger 9 gelagert sind.

Fig. 8 zeigt eine Chirurgieroboteranordnung nach einer weiteren Ausführung der vorliegenden Erfindung in Fig. 7 entsprechender Darstellung. Einander entsprechende Merkmale sind durch identische Bezugszeichen bezeichnet, so dass nachfolgend nur auf Unterschiede zur Ausführung der Fig. 7 eingegangen und im Übrigen auf deren Beschreibung Bezug genommen wird.

In der Ausführung der Fig. 8 sind die Positioniervorrichtungsbasen 1 der drei Positioniervorrichtungen unabhängig voneinander in der vorstehend beschriebenen Weise durch separate Hubsäulen 19 linear beweglich und drehbar an separaten Trägern 9 gelagert.

Fig. 9 zeigt eine Chirurgieroboteranordnung nach einer weiteren Ausführung der vorliegenden Erfindung in Fig. 7, 8 entsprechender Darstellung. Einander entsprechende Merkmale sind durch identische Bezugszeichen bezeichnet, so dass nachfolgend nur auf Unterschiede zur Ausführung der Fig. 7, 8 eingegangen und im Übrigen auf deren Beschreibung Bezug genommen wird.

In der Ausführung der Fig. 9 sind die separaten Hubsäulen 19 jeweils durch eine horizontale Linearachse 19.1, welche senkrecht zu der Achse des Gelenks 2 der Kinematik ist, mit dem die Positioniervorrichtungsbasis 1 verbunden ist, zusätzlich linear beweglich. Dafür entfällt bei der Ausführung der Fig. 9 die weitere Linearachse 17, die Chirurgieroboterbasis 11 ist über das weitere Drehgelenk 17' direkt an dem Positioniervorrichtungsflansch 7 angelenkt.

Fig. 10 zeigt eine Chirurgieroboteranordnung nach einer weiteren Ausführung der vorliegenden Erfindung in Fig. 7, 8, 9 entsprechender Darstellung. Einander entsprechende Merkmale sind durch identische Bezugszeichen bezeichnet, so dass nachfolgend nur auf Unterschiede zur Ausführung der Fig. 9 eingegangen und im Übrigen auf die vorhergehende Beschreibung Bezug genommen wird.

In der Ausführung der Fig. 10 sind die separaten Hubsäulen 19 jeweils durch eine horizontale Linear-Drehgelenk 19.2, dessen Achse senkrecht zu der Achse des Gelenks 2 der Kinematik ist, mit dem die Positioniervorrichtungsbasis 1 verbunden ist, sowohl linear beweglich als auch dreh- bzw. kippbar.

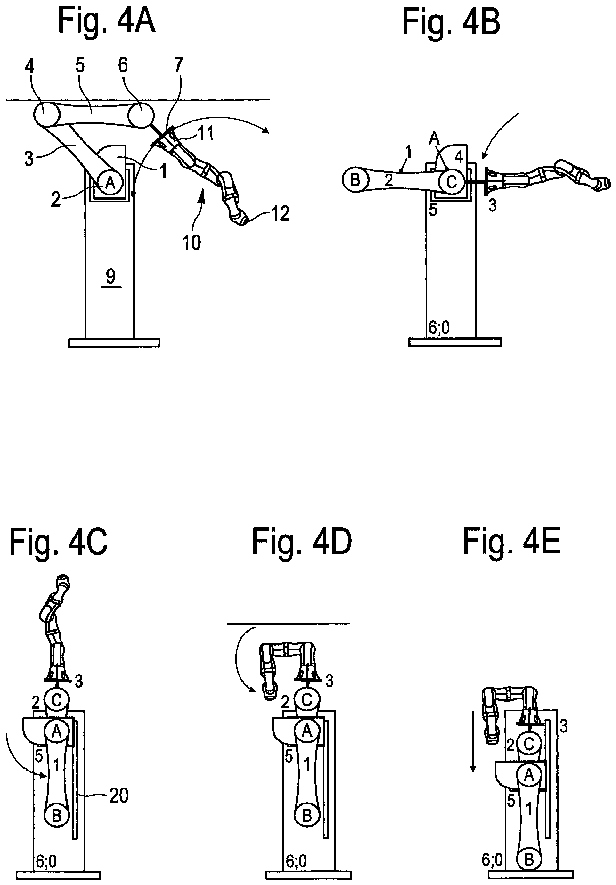

Mit Bezug auf die Figurenfolge Fig. 4A - 4E wird nun ein Lagervorgang einer Chirurgieroboteranordnung nach einer Ausführung der vorliegenden Erfindung bzw. eine hierzu entsprechend ausgebildete Chirurgieroboteranordnung bzw. Positioniervorrichtung näher erläutert. Diese kann insbesondere einer der vorstehend mit Bezug auf Fig. 1-3, 5-10 erläuterten entsprechen, so dass auf deren Beschreibung Bezug genommen und nachfolgend nur auf Unterschiede eingegangen wird.

In der Ausführung der Fig. 4A - 4E weist der Träger 9 ein Gehäuse auf, in das die Positioniervorrichtungsbasis 1, die hierzu gefaltete Kinematik 2-6 und der Positioniervorrichtungsflansch 7 einfahrbar ist.

Dazu werden zunächst die Glieder 3, 5 der Kinematik in eine Lagerstellung zusammengefaltet, in der diese beiden Glieder gegensinnig parallel zueinander sind (Fig. 4A → Fig. 4B). Anschließend wird die Positioniervorrichtungsbasis 1, die hierzu drehbar an einem Schlitten 20 des Trägers 9 angeordnet ist, verschwenkt, wodurch die Glieder 3, 5 in das Gehäuse einschwenken (Fig. 4B → Fig. 4C). Optional kann der Chirurgieroboter 10 in einer kompakte Lagerpose gesteuert werden (Fig. 4C → Fig. 4D). In einem weiteren Schritt wird die Positioniervorrichtung mittels des Schlittens 20 in das Gehäuse abgesenkt.

Zum Positionieren der Chirurgieroboter 10 der Chirurgieroboteranordnung gem. Fig. 7 bis 10 werden die Chirurgieroboter zum manuellen Verstellen des Positioniervorrichtungsflanschs 7 zunächst in eine vorgegebene, in Fig. 8 bis 10 angedeutete Positionierungspose gesteuert, in der sie eine vorteilhafte Manipulierbar- bzw. Beweglichkeit aufweisen. Die Positionierungspose wird auf Basis einer durchzuführenden Operation aus mehreren vorgegebenen Posen ausgewählt.

Nachdem die Positioniervorrichtungen in der manuell eingestellten Lage fixiert wurden, wird ein positioniervorrichtungsbasis-, träger- oder umgebungsfestes Kalibriermittel (nicht dargestellt) mit einem ausgezeichneten Punkt des Chirurgieroboterflanschs 12 oder -endeffektors 13 mehreren voneinander verschiedenen Posen des Chirurgieroboters 10 angefahren und die zugehörigen Gelenkkoordinaten des Chirurgieroboters ausgewertet. Auf diese Weise kann ein Bezugssystem einer Steuerung des Chirurgieroboters kalibriert und so eine Vor- bzw. Umpositionierung seiner Chirurgieroboterbasis kompensiert werden.

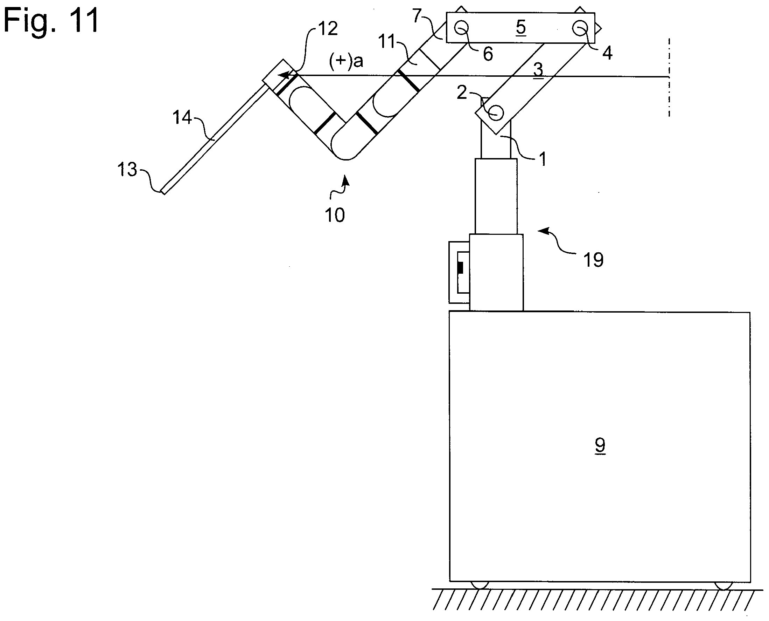

Fig. 11 zeigt eine Manipulatoranordnung mit einem Trägersystem nach einer Ausführung der vorliegenden Erfindung in einer Seitansicht. Mit den vorstehenden Ausführungen übereinstimmende Merkmale sind durch identische Bezugszeichen identifiziert, so dass auf deren Beschreibung Bezug genommen wird. Wie bereits vorstehend betont, sei an dieser Stelle nochmals darauf hingewiesen, dass eine Positioniervorrichtung, eine Manipulator-, insbesondere Roboteranordnung, ein Verfahren zum Positionieren eines Roboters bzw. ein Trägersystem nach einem Aspekt der vorliegenden Erfindung am Beispiel von Chirurgierobotern erläutert wird, wobei in einer Abwandlung gleichermaßen an deren Stelle auch anderer Roboter, insbesondere Industrie- oder Serviceroboter, sein können.

Man erkennt den bereits vorstehend erläuterten siebenachsigen Chirurgieroboter 10 mit dem an dem Roboterflansch 12 befestigten Instrumentenschaft 14 und dem chirurgischen Endeffektor 13. Die Chirurgieroboterbasis 11 ist an dem Positioniervorrichtungsflansch 7 der Positioniervorrichtung befestigt, die über die Kinematik 2-6 mit der Positioniervorrichtungsbasis 1 verbunden ist, welche über die Hubsäule 12 an dem Träger 9 verstellbar gelagert ist. Wie bereits betont, stellt eine Chirurgieroboteranordnung eine vorteilhafte Verwendung des nachfolgend mit Bezug auf Fig. 11-14 erläuterten Trägersystems dar, dieses ist jedoch nicht darauf beschränkt, sondern kann gleichermaßen mit Industrie- oder Servicemanipulatoren, insbesondere -robotern, verwendet werden.

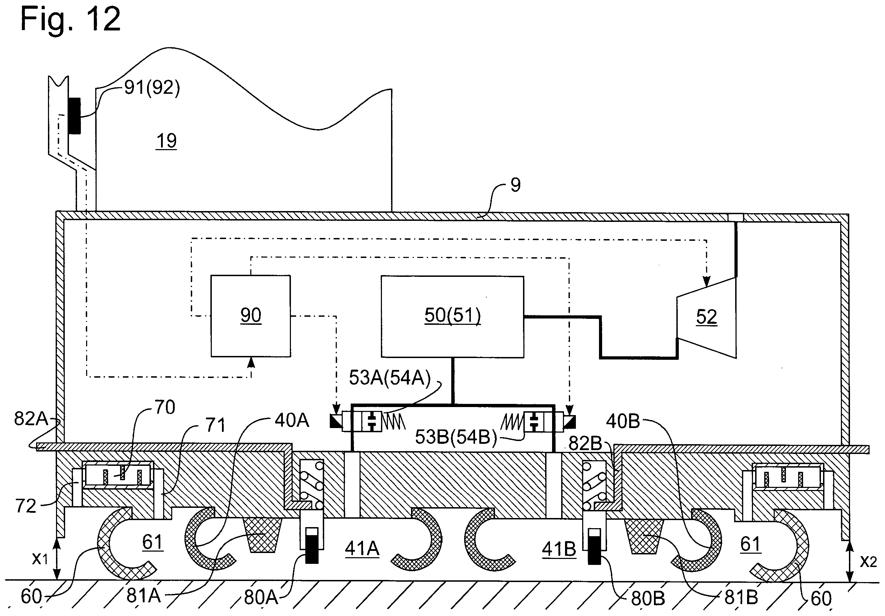

Fig. 12 zeigt einen Schnitt durch einen Teil des Trägers 9. An diesem ist eine Luftkammeranordnung angeordnet, die im Ausführungsbeispiel der Fig. 12 einen von einer Druckschürze 40A umkleideten Druckraum 41A und einen von einer weiteren Druckschürze 40B umkleideten Druckraum 41B aufweist, die zur Ausbildung eines Luftkissens unabhängig voneinander über Steuer- bzw. Stellventile 53A bzw. 53B mit Überdruckluft von einer Überdruckluftversorgung versorgbar sind. Die Überdruckluftversorgung weist im Ausführungsbeispiel der Fig. 12 eine trägerseitige Überdruckluftquelle mit einer Strömungsmaschine in Form eines Kompressors 52 und einem Überdruckspeicher in Form eines Überdruckbehälters 50 auf, der von dem Kompressor aufladbar ist bzw. aufgeladen wird.

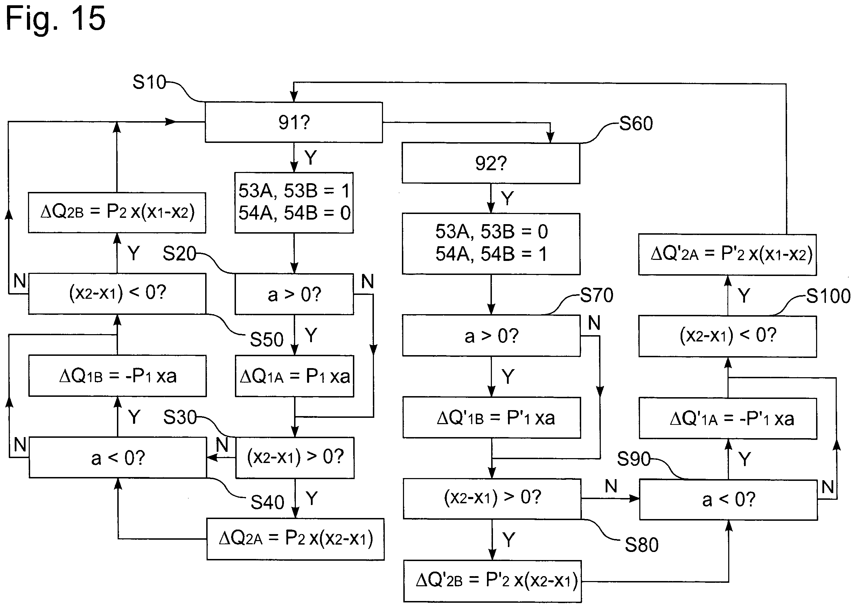

Zum Bewegen des Trägers 9 betätigt der Anwender eine Bedieneinrichtung in Form eines Betätigungsknopfes 91, der dies an einen Controller 90 übermittelt. Dieser öffnet entsprechend die Ventile 53A, 53B, so dass die Druckräume 41A, 41B mit Überdrucklauf aus dem Überdruckbehälter 50 der Überdruckluftversorgung versorgt bzw. beschickt und der hierdurch luftkissengetragene Träger 9 manuell bewegbar ist. In nachfolgend mit Bezug auf Fig. 15 erläuterter Weise regelt der Controller dabei die Stellung der Ventile. Zusätzlich oder alternativ kann der Controller auch den Kompressor steuern.

Die dabei durch einen Spalt zwischen Druckschürzen 40A, 40B und dem Boden strömende Überdruckluft kann eine unerwünschte Geräuschemission und Staubaufwirbelung verursachen. Daher umkleidet eine gemeinsame Schallschürze 60 einen Schallraum 61, der die Druckräume 41A, 41B umgibt. Die Schallschürze 60 ist elastisch gegen den Boden vorgespannt und weist eine bodenseitige Dichtlippe auf.

Der Schallraum 61 kommuniziert über eine Passage 71 mit einem trägerseitigen Schalldämpfermittel in Form eines Absorptionsschalldämpfers 70 zum Dämpfen von Luftschall in dem Schallraum. Die solcherart schallgedämpfte Luft tritt über eine Passage 72 in die Umgebung aus.

Zum Fixieren des Trägers 9 betätigt der Anwender einen weiteren Betätigungsknopf 92, der dies an einen Controller 90 übermittelt. Dieser schließt entsprechend die Ventile 53A, 53B und sperrt die Druckräume 41A, 41B von dem Überdruckbehälter 50 der Überdruckluftversorgung ab. Stattdessen öffnet der Controller nun analog Stellventile 54A, 54B, die die Druckräume 41A, 41B mit Unterdruck aus einem Unterdruckspeicher in Form eines Unterdruckbehälters 51 einer trägerseitigen Unterdruckluftquelle einer Unterdruckluftversorgung versorgt, der durch den reversibel betriebenen Kompressor 52 evakuierbar ist. Hierdurch wird in den Druckräumen 41A, 41B ein Unterdruck ausgebildet, der den Träger 9 am Boden fixiert. In nachfolgend mit Bezug auf Fig. 15 erläuterter Weise regelt der Controller dabei die Stellung der Ventile. Zusätzlich oder alternativ kann der Controller auch den Kompressor steuern.

Dabei stützt sich der Träger durch eine Stützanordnung mit drei oder vier elastischen Stützen in den Druckräumen 41A bzw. 41B ab, von denen im Schnitt der Fig. 12 zwei Stützen 81A, 81B sichtbar sind.

Das Trägersystem weist weiter eine stationär an dem Träger angeordnete Rollenanordnung mit wenigstens drei in den Druckräumen 41A, 41B angeordneten Transportrollen auf, von denen im Schnitt der Fig. 12 zwei Transportrollen 80A bzw. 80B sichtbar sind. Diese werden im Ausführungsbeispiel durch Druckfedern aus einer Lager- in eine Betriebsstellung vorgespannt, in der sie bodenseitig über die Stützen 81A, 81B überstehen. In dieser Betriebsstellung können die Transportrollen durch eine Verriegelung 82A, 82B blockiert werden, die ein Einfahren in die Lagerstellung sperrt. Bei gelöster Verriegelung fahren die Transprotrollen hingegen in ihre Lagerstellung ein, so dass der Träger auf den Stützen 81A, 81B aufsitzt, wenn das Luftkissen nicht ausgebildet ist. Bei ausgebildetem Luftkissen sind hingegen, wie in Fig. 12 gezeigt, sowohl die Stützen als auch die Transportrollen vom Boden beabstandet.

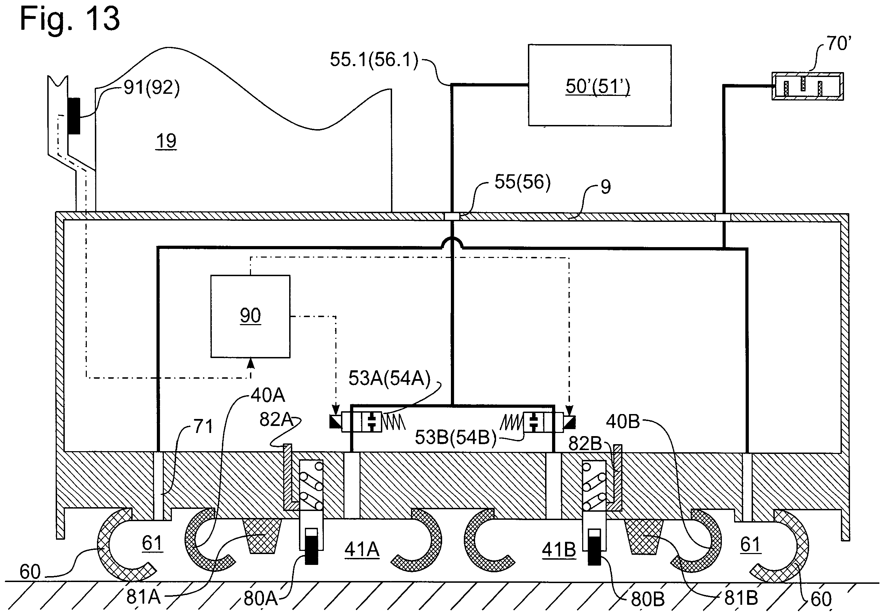

Fig. 13 zeigt in Fig. 12 entsprechender Weise ein Trägersystem nach einer weiteren Ausführung der vorliegenden Erfindung. Mit der vorstehend erläuterten Ausführung übereinstimmende Merkmale sind durch identische Bezugszeichen identifiziert, so dass auf die vorstehende Beschreibung Bezug genommen und nachfolgend nur auf Unterschiede eingegangen wird.

In der Ausführung der Fig. 13 sind die Überdruckluftquelle in Form eines Überdruckspeichers bzw. -behälters 50', die Unterdruckluftquelle in Form eines Unterdruckspeichers bzw. -behälters 51' sowie das Schalldämpfermittel in Form eines Absorptionsschalldämpfers 70' trägerextern. Entsprechend weist die Überdruckluftversorgung einen trägerseitigen Luftleitungsanschluss 55, der über eine flexible Leitung 55.1 lösbar mit dem trägerfernen Überdruckbehälter 50' verbunden ist, und die Unterdruckluftversorgung einen trägerseitigen Luftleitungsanschluss 56 auf, der über eine flexible Leitung 56.1 lösbar mit dem trägerfernen Unterdruckbehälter 51' verbunden ist. Der trägerferne Absorptionsschalldämpfer 70' kommuniziert mit dem Schallraum 61 ebenfalls über eine lösbar mit dem Träger 3 verbundene flexible Luftleitung und leitet Überdruckluft auf dem Schallraum 61 trägerfern und schallgedämpft in die Umgebung aus.

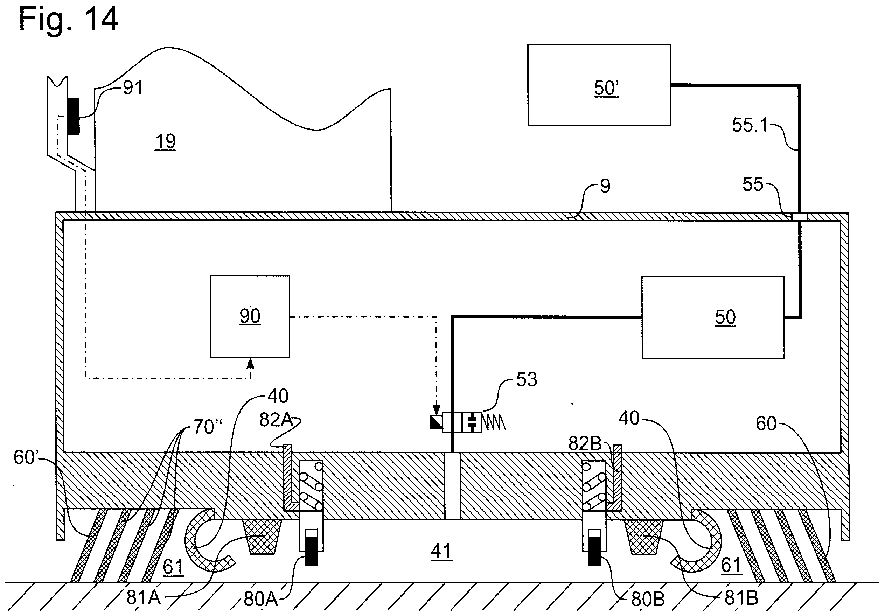

Fig. 14 zeigt in Fig. 12, 13 entsprechender Weise ein Trägersystem nach einer weiteren Ausführung der vorliegenden Erfindung. Mit der vorstehend erläuterten Ausführung übereinstimmende Merkmale sind wiederum durch identische Bezugszeichen identifiziert, so dass auf die vorstehende Beschreibung Bezug genommen und nachfolgend nur auf Unterschiede eingegangen wird.

In der Ausführung der Fig. 14 ist das Schalldämpfermittel in Form mehrerer konzentrischen Lamellenreihen 70" in dem Schallraum 61 angeordnet, die Schallschürze 60' ist ebenfalls als (äußerste) Lamellenreihe ausgebildet.

Zudem ist der trägerseitige Überdruckbehälter 50, der insbesondere als Puffer fungiert, einerseits über den trägerseitigen Luftleitungsanschluss 55 und die flexible Leitung 55.1 lösbar mit dem trägerfernen Überdruckbehälter 50' verbunden und andererseits durch ein Stellventil 53 mit Überdruckluft aus dem von der Druckschürze 40 umkleideten einzigen Druckraum 41 versorgbar, indem der Controller 90 infolge einer entsprechenden Betätigung des Betätigungsknopfes 91 dieses Ventil öffnet, insbesondere zur Regelung des vertikalen Bodenabstandes stärker und/oder länger öffnet oder schließt.

Nachfolgend wird exemplarisch anhand der Fig, 15 ein Verfahren zum Bewegen und/oder Fixieren des Trägers 9 des Trägersystems der Fig. 13 erläutert, das dessen Controller 90 durchführt.

Solange der Anwender den Betätigungsknopf 91 betätigt (S10: "Y"), öffnet der Controller 90 die Ventile 53A, 53B, so dass die Druckräume 41A, 41B mit Überdruckluft aus dem Überdruckluftbehälter 50 versorgt werden (S20).

Dabei erfasst der Controller 90 über Erfassungsmittel in Form von Gelenksensoren (nicht dargestellt) eine Pose des Manipulators 10 und ermittelt hieraus als Auslage den gerichteten bzw. vorzeichenbehafteten horizontalen Abstand a seines Flansches 12 von einer kippmomentfreien Referenzkonfiguration (vgl. Fig. 11). Desweiteren erfasst der Controller 90 über Abstandssensoren an den bodennahen Ecken des Trägers (nicht dargestellt) eine gerichteten bzw. vorzeichenbehafteten Lage bzw. Auslenkung gegen die Horizontale x₂-x₁ des Trägers 9 (vgl. Fig. 11).

Der Controller 90 vergrößert durch entsprechende Ansteuerung des Ventils 53A die Luftzufuhr Q zum Druckraum 41A um ΔQ_{1A} gemäß der Regelvorschrift ΔQ_{1A} = P_{1.a} mit dem konstanten positiven Proportionalitätsfaktor P₁, sofern die Auslage a positiv bzw. zum Druckraum 41A gerichtet ist (S20: "Y"), und um ΔQ_{2A}, gemäß der Regelvorschrift ΔQ_{2A} = P₂·(x₂-x₁) mit dem konstanten positiven Proportionalitätsfaktor P₂, sofern die Auslenkung (x₂-x₁) positiv bzw. zum Druckraum 41A gerichtet ist (S30: "Y"). In analoger Weise vergrößert der Controller 90 durch entsprechende Ansteuerung des Ventils 53B die Luftzufuhr Q zum Druckraum 41B um ΔQ_{1B} gemäß der Regelvorschrift ΔQ_{1B} = -P₁·a, sofern die Auslage a negativ bzw. zum Druckraum 41B gerichtet ist (S40: "Y"), und um ΔQ_{2B} gemäß der Regelvorschrift ΔQ_{2B} = P₂·(x₁-x₂), sofern die Auslenkung (x₂-x₁) negativ bzw. zum Druckraum 41B gerichtet ist (S50: "Y").

Hat der Anwender hingegen durch Betätigung des Betätigungsknopfes 92 eine Fixierung am Boden kommandiert (S60: "Y"), schließt der Controller 90 die Ventile 53A, 53B und öffnet die Ventile 54A, 54B, so dass die Druckräume 41A, 41B nun mit Unterdruck aus dem Unterdruckluftbehälter 51 versorgt werden.

Der Controller 90 vergrößert durch entsprechende Ansteuerung des Ventils 54B die Luftabfuhr Q' aus dem Druckraum 41B um ΔQ'_{1B} gemäß der Regelvorschrift ΔQ'_{1B} = P'₁·a mit dem konstanten positiven Proportionalitätsfaktor P'₁, sofern die Auslage a positiv bzw. zum Druckraum 41A gerichtet ist (S70: "Y"), und um ΔQ'_{2B} gemäß der Regelvorschrift ΔQ'_{2B} = P'₂·(x₂-x₁) mit dem konstanten positiven Proportionalitätsfaktor P'₂, sofern die Auslenkung (x₂-x₁) positiv bzw. zum Druckraum 41A gerichtet ist (S80: "Y"). In analoger Weise vergrößert der Controller 90 durch entsprechende Ansteuerung des Ventils 54A die Luftabfuhr Q' aus dem Druckraum 41A um ΔQ'_{1A} gemäß der Regelvorschrift ΔQ'_{1A} = -P'₁·a, sofern die Auslage a negativ bzw. zum Druckraum 41B gerichtet ist (S90: "Y"), und um ΔQ'_{2A} gemäß der Regelvorschrift ΔQ'_{2A} = P'₂·(x₁-x₂), sofern die Auslenkung (x₂-x₁) negativ bzw. zum Druckraum 41B gerichtet ist (S100: "Y").

Hierdurch erhöht der Controller 90 auf Basis der erfassten Pose des Manipulators 10 und der erfassten Lage des Trägers 9 den Überdruck auf einer Seite, über die ein Kippmoment, das durch eine Auslage a des Manipulators induziert wird und sich in einer Auslenkung des Trägers gegen die Horizontale auswirkt, das Trägersystem zu kippen sucht. Analog erhöht der Controller 90 auf Basis der erfassten Pose des Manipulators 10 und der erfassten Lage des Trägers 9 den Unterdruck auf einer Seite des Trägersystems, die ein Kippmoment, das durch eine Auslage a des Manipulators induziert wird und sich in einer Auslenkung des Trägers gegen die Horizontale auswirkt, anzuheben sucht.

**Bezugszeichenliste**

| | |
|---|---|
| 1 | Positioniervorrichtungsbasis |
| 2, 4, 6; 24 | Gelenke der Kinematik |
| 3, 5; 35 | Glieder der Kinematik |
| 7 | Positioniervorrichtungsflansch |
| 8; 8' | Druckfeder (Spannmittel) |
| 9 | Träger |
| 10 | Chirurgieroboter |
| 11 | Chirurgieroboterbasis |
| 12 | Chirurgieroboterflansch |
| 13 | Endeffektor |
| 14 | Instrumentenschaft |
| 17 | weiteres Lineargelenk |
| 17' | weiteres Drehgelenk |
| 19 | drehbare Hubsäule |
| 19.1 | Linearachse |
| 19.2 | Linear-Drehachse |
| 20 | Schlitten |
| 100; 101; 102 | mechanische Zwangskopplung |
| 201; 203; 204; 206 | Drehgelenk |
| 202/205 | erste/zweite Koppelstange |
| 301/305/ 303/307 | erste/zweite/dritte/vierte Synchronscheibe |
| 304/306 | erstes/zweites Zugmittel |
| V; V' | virtueller Drehpunkt |
| | |
| 40; 40A, 40B | Druckraum |
| 41; 41A, 41B | Druckschürze |
| 50; 50' | Überdruckluftbehälter |
| 51; 51' | Unterdruckluftbehälter |
| 52 | Kompressor |
| 53; 53A, 53B, 54A, 54B | Stellventil |
| 55, 56 | Luftleitungsanschluss |
| 55.1, 56.1 | Luftleitung |
| 60; 60' | Schallschürze |
| 61 | Schallraum |
| 70; 70'; 70" | Schalldämpfermittel |
| 71, 72 | Passage |
| 80A, 80B | Trasportrolle |
| 81A, 81B | Stütze |
| 90 | Controller |
| 91, 92 | Betätigungsknopf |
| 82A, 82B | Verriegelung |

## Patentansprüche

1. Positioniervorrichtung für einen Roboter (10) mit einem, insbesondere chirurgischen, Endeffektor (13), die eine Basis (1) und einen Flansch (7) aufweist, an dem der Roboter befestigbar ist, wobei der Flansch durch eine Kinematik, die wenigstens zwei Gelenke (2, 4, 6) aufweist, mit der Basis verbunden ist, durch die der Flansch von einer ersten Position relativ zu der Basis, insbesondere wenigstens im Wesentlichen auf einer Kreisbahn oder Geraden, in eine hiervon beabstandete zweite Position relativ zu der Basis verstellbar ist, **gekennzeichnet durch** ein Orientierungsmittel (100, 101; 102) zum Umorientieren des Flansches infolge einer Verstellung von der ersten in die zweite Position von einer ersten Orientierung in der ersten Position in eine, insbesondere gegenüber der ersten Orientierung um wenigstens 75° um eine Referenzachse verdrehte, zweite Orientierung in der zweiten Position.

2. Positioniervorrichtung nach dem vorhergehenden Anspruch, **dadurch gekennzeichnet, dass** der Flansch durch die Kinematik in wenigstens eine Zwischenposition zwischen der ersten und zweiten Position relativ zu der Basis verstellbar ist, wobei das Orientierungsmittel zum Umorientieren des Flansches infolge einer Verstellung von der ersten oder zweiten Position in die Zwischenposition in eine, insbesondere gegenüber der ersten und/oder zweiten Orientierung um wenigstens 25° und/oder höchstens 60° um die Referenzachse verdrehte, Zwischenorientierung in der Zwischenposition eingerichtet ist.

3. Positioniervorrichtung nach einem der vorhergehenden Ansprüche, **dadurch gekennzeichnet, dass** die Kinematik wenigstens ein Drehgelenk, insbesondere wenigstens drei, insbesondere seriell angeordnete, Drehgelenke (2, 4, 6), insbesondere mit parallelen Drehachsen, und/oder wenigstens ein Lineargelenk (24) aufweist.

4. Positioniervorrichtung nach einem der vorhergehenden Ansprüche, **dadurch gekennzeichnet, dass** das Orientierungsmittel wenigstens eine, insbesondere lösbare, mechanische Zwangskopplung (100, 101; 102) von zwei Gliedern (1, 3, 5, 7) der Positioniervorrichtung aufweist, die durch wenigstens ein Gelenk der Kinematik miteinander verbunden sind.

5. Positioniervorrichtung nach dem vorhergehenden Anspruch, **dadurch gekennzeichnet, dass** die mechanische Zwangskopplung ein Getriebe, insbesondere ein Räder-, Zugmittel- (301, 303, 304, 305, 306, 307), Schrauben-, Kurven-, Koppel-, insbesondere Schubstangen- oder Koppelstangen- (201, 202, 203, 204, 205, 206) oder hydraulisches Getriebe, aufweist.

6. Positioniervorrichtung nach einem der vorhergehenden Ansprüche, **dadurch gekennzeichnet, dass** die Basis und der Flansch durch drei seriell angeordnete Drehgelenke (2, 4, 6) mit parallelen Drehachsen verbunden sind, wobei durch das Orientierungsmittel eine Viergelenk-, insbesondere Parallelogrammführung gebildet ist, die die drei seriell angeordneten Drehgelenke der Kinematik und einen relativ zu der Basis ortsfesten virtuellen Drehpunkt (V) mit parallelen Drehachsen aufweist.

7. Positioniervorrichtung nach einem der vorhergehenden Ansprüche, **dadurch gekennzeichnet, dass** die Basis und der Flansch durch ein Lineargelenk (24) und ein damit verbundenes Drehgelenk (6) verbunden sind, wobei das Orientierungsmittel eine mechanische Zwangskopplung (102) der Basis und des Flansches aufweist.

8. Positioniervorrichtung nach einem der vorhergehenden Ansprüche, **dadurch gekennzeichnet, dass** wenigstens ein Gelenk, insbesondere alle Gelenke (2, 4, 6), der Kinematik eine Verriegelung zum, insbesondere mechanischen und/oder manuellen und/oder aktuierten, Blockieren des jeweiligen Gelenks, insbesondere zusammen mit und/oder unabhängig von wenigstens einem anderen Gelenk der Kinematik, aufweisen.

9. Positioniervorrichtung nach einem der vorhergehenden Ansprüche, **gekennzeichnet durch** ein, insbesondere mechanisches und/oder pneumatisches, Spannmittel (8; 8'), das zwei durch wenigstens ein Gelenk (2; 24, 6) der Kinematik miteinander verbundene Glieder (1, 3; 1, 35) der Positioniervorrichtung miteinander fesselt und durch eine Bewegung der Positioniervorrichtung in eine Lage, in der die Positioniervorrichtung eine niedrigere Lageenergie aufweist, stärker gespannt wird.

10. Positioniervorrichtung nach einem der vorhergehenden Ansprüche, **dadurch gekennzeichnet, dass** die Positioniervorrichtung wenigstens zwei Glieder (3, 5) aufweist, die durch wenigstens ein Gelenk (4) der Kinematik miteinander verbunden und in eine Lagerstellung zusammenfaltbar sind, in der die beiden Glieder, wenigstens im Wesentlichen, gegensinnig parallel zueinander sind.

11. Positioniervorrichtung nach einem der vorhergehenden Ansprüche, **gekennzeichnet durch** einen Träger (9), an dem die Basis (1) ein- oder mehrachsig linear beweglich und/oder drehbar gelagert ist.

12. Roboteranordnung, mit:
einem Roboter (10) mit einem, insbesondere chirurgischen, Endeffektor (13), und einer Positioniervorrichtung nach einem der vorhergehenden Ansprüche, wobei der Roboter, insbesondere lösbar, an dem Flansch (7) befestigt ist.

13. Roboteranordnung nach dem vorhergehenden Anspruch, **dadurch gekennzeichnet, dass** der Roboter starr oder linear beweglich (17) und/oder drehbar (17') an dem Flansch (7) befestigt ist.

14. Verfahren zum Positionieren des Roboters (10) einer Roboteranordnung mit einem Roboter mit einem, insbesondere chirurgischen, Endeffektor (13) und einer Positioniervorrichtung nach einem der vorhergehenden Ansprüche 1 bis 11, wobei der Roboter, insbesondere lösbar, an dem Flansch (7) befestigt ist, insbesondere starr oder linear beweglich (17) und/oder drehbar (17') an dem Flansch (7) befestigt ist, **dadurch gekennzeichnet, dass** der Roboter zum, insbesondere manuellen, Einstellen der Positioniervorrichtung in eine vorgegebene, insbesondere aus mehreren vorgegebenen Posen ausgewählte, Positionierungspose gesteuert wird und/oder nach einem Fixieren der Positioniervorrichtung ein Kalibriermittel mit einem roboterfesten Referenzpunkt in wenigstens einer Pose des Roboters angefahren wird.

## Claims

1. A positioning device for a robot (10) comprising an end effector (13), in particular a surgical end effector (13), which positioning device has a base (1) and a flange (7) to which the robot can be secured, wherein the flange is connected to the base by a kinematic system which comprises at least two joints (2, 4, 6), by means of which kinematic system the flange can be adjusted from a first position relative to the base, in particular at least substantially on a circular path or on a straight line, to a second position relative to the base, the second position being spaced apart from the first position, **characterised by** an orientation means (100, 101; 102) for reorienting the flange, as a consequence of an adjustment from the first position into the second position, from a first orientation in the first position into a second orientation in the second position, the second orientation in particular being rotated about a reference axis by at least 75° with respect to the first orientation.

2. The positioning device according to the preceding claim, **characterised in that** the flange is adjustable relative to the base, by means the kinematic system, into at least one intermediate position between the first position and the second position, wherein the orientation means is set up to reorient the flange, as a consequence of an adjustment from the first position or the second position into the intermediate position, into an intermediate orientation in the intermediate position, the intermediate orientation in particular being rotated about the reference axis by at least 25° and / or at most 60° with respect to the first orientation and / or the second orientation.

3. The positioning device according to any one of the preceding claims, **characterised in that** the kinematic system comprises at least one rotary joint, in particular at least three rotary joints (2, 4, 6), in particular at least three rotary joints (2, 4, 6) arranged in series, in particular with parallel axes of rotation, and / or at least one linear joint (24).

4. The positioning device according to any one of the preceding claims, **characterised in that** the orientation means comprises at least one mechanical positive coupling (100, 101; 102) of two links (1, 3, 5, 7) of the positioning device, in particular a releasable mechanical positive coupling (100, 101; 102) of two links (1, 3, 5, 7) of the positioning device, which are connected to one another by at least one joint of the kinematic system.

5. The positioning device according to the preceding claim, **characterised in that** the mechanical positive coupling comprises a transmission, in particular a gear train, a traction gear (301, 303, 304, 305, 306, 307), a worm gear, a cam gear, a coupling gear, in particular a connecting rod transmission or a coupling rod transmission (201, 202, 203, 304, 205, 206), or a hydraulic transmission.

6. The positioning device according to any one of the preceding claims, **characterised in that** the base and the flange are connected by means of three rotary joints (2, 4, 6) which are arranged in series and which have parallel axes of rotation, wherein a four-bar guide mechanism, in particular a parallelogram guide mechanism, is formed by means of the orientation means, which guide mechanism comprises the three rotary joints of the kinematic system, which three rotary joints are arranged in series, and a virtual point of rotation (V), which is stationary relative to the base, with parallel axes or rotation.

7. The positioning device according to any one of the preceding claims, **characterised in that** the base and the flange are connected by a linear joint (24) and a rotary joint (6) connected thereto, wherein the orientation means comprises a mechanical positive coupling (102) of the base and the flange.

8. The positioning device according to any one of the preceding claims, **characterised in that** at least one joint, in particular all joints (2, 4, 6) of the kinematic system have a locking mechanism for locking of the respective joint, in particular in a mechanical and / or manual and / or actuated manner, in particular together with, and / or independent from, at least one other joint of the kinematic system.

9. The positioning device according to any one of the preceding claims, **characterised by** a biasing means (8; 8'), in particular a mechanical and / or pneumatic biasing means (8; 8'), which binds two links (1, 3; 1, 35) of the positioning device, which two links (1, 3; 1, 35) are connected to one another by at least one joint (2; 24, 6) of the kinematic system, and which biasing means (8; 8') is more strongly biased by a movement of the positioning device into a position and orientation in which the positioning device has a lower position and orientation energy.

10. The positioning device according to any one of the preceding claims, **characterised in that** the positioning device comprises at least two links (3, 5) which are connected to one another by means of at least one joint (4) of the kinematic system and which can be folded together into a storage position in which the two links are at least substantially parallel to one another in opposite directions.

11. The positioning device according to any one of the preceding claims, **characterised by** a carrier (9) on which the base (1) is mounted to be linearly moveable and / or rotatable in terms of one or multiple axes.

12. A robot arrangement comprising:
a robot (10) with an end effector (13), in particular a surgical end effector (13), and a positioning device according to any one of the preceding claims, wherein the robot is secured to the flange (7), in particular in a releasable manner.

13. The robot arrangement according to the preceding claim, **characterised in that** the robot is secured rigidly, or linearly moveably (17), and / or rotatably (17') to the flange (7).

14. A method of positioning the robot (10) of a robot arrangement comprising a robot with an end effector (13), in particular a surgical end effector (13), and a positioning device according to any one of the preceding claims 1 to 11, wherein the robot is secured to the flange (7), in particular in a releasable manner, in particular secured rigidly, or linearly moveably (17) and / or rotatably (17') to the flange (7), **characterised in that**, for the purpose of adjusting the positioning device, in particular for the purpose of manually adjusting the positioning device, the robot is steered into a predetermined positioning pose, in particular into a predetermined positioning pose which is selected from a plurality of predetermined poses, and / or, after the positioning device has been fixed, a calibration means with a reference point which is fixed with respect to the robot is approached in at least one pose of the robot.

## Revendications

1. Dispositif de positionnement pour un robot (10) avec un effecteur terminal (13), en particulier chirurgical, qui présente une base (1) et une bride (7), sur laquelle le robot peut être fixé, dans lequel la bride est reliée à la base par une cinématique, qui présente au moins deux articulations (2, 4, 6), par laquelle la bride peut être déplacée d'une première position par rapport à la base, en particulier au moins sensiblement sur une trajectoire circulaire ou ligne droite, à une deuxième position espacée de celle-ci par rapport à la base, **caractérisé par** un moyen d'orientation (100, 101 ; 102) pour la réorientation de la bride suite à un déplacement de la première à la deuxième position d'une première orientation dans la première position à une deuxième orientation, en particulier tournée par rapport à la première orientation d'au moins 75° autour d'un axe de référence, dans la deuxième position.

2. Dispositif de positionnement selon la revendication précédente, **caractérisé en ce que** la bride peut être déplacée par la cinématique dans au moins une position intermédiaire entre la première et deuxième position par rapport à la base, dans lequel le moyen d'orientation est aménagé pour la réorientation de la bride suite à un déplacement de la première ou deuxième position à la position intermédiaire dans une orientation intermédiaire, en particulier tournée par rapport à la première et/ou deuxième orientation d'au moins 25° et/ou de maximum 60° autour de l'axe de référence, dans la position intermédiaire.

3. Dispositif de positionnement selon l'une quelconque des revendications précédentes, **caractérisé en ce que** la cinématique présente au moins une articulation rotative, en particulier au moins trois articulations rotatives (2, 4, 6), agencées en particulier en série, en particulier avec des axes de rotation parallèles, et/ou au moins une articulation linéaire (24).

4. Dispositif de positionnement selon l'une quelconque des revendications précédentes, **caractérisé en ce que** le moyen d'orientation présente au moins un accouplement forcé mécanique (100, 101 ; 102), en particulier amovible, de deux maillons (1, 3, 5, 7) du dispositif de positionnement, qui sont reliés l'un à l'autre par au moins une articulation de la cinématique.

5. Dispositif de positionnement selon la revendication précédente, **caractérisé en ce que** l'accouplement forcé mécanique présente une transmission, en particulier un train d'engrenages, une transmission à moyen de traction (301, 303, 304, 305, 306, 307), une transmission à vis, une transmission à cames, une transmission de couplage, en particulier par tiges de poussée ou tiges de couplage (201, 202, 203, 204, 205, 206) ou une transmission hydraulique.

6. Dispositif de positionnement selon l'une quelconque des revendications précédentes, **caractérisé en ce que** la base et la bride sont reliées par trois articulations rotatives (2, 4, 6) agencées en série avec des axes de rotation parallèles, dans lequel un guidage par quadrilatère articulé, en particulier guidage par parallélogramme, qui présente les trois articulations rotatives agencées en série de la cinématique et un point de rotation virtuel (V) stationnaire par rapport à la base avec des axes de rotation parallèles, est formé par le moyen d'orientation.

7. Dispositif de positionnement selon l'une quelconque des revendications précédentes, **caractérisé en ce que** la base et la bride sont reliées par une articulation linéaire (24) et une articulation rotative (6) reliée à celle-ci, dans lequel le moyen d'orientation présente un accouplement forcé mécanique (102) de la base et de la bride.

8. Dispositif de positionnement selon l'une quelconque des revendications précédentes, **caractérisé en ce qu'**au moins une articulation, en particulier toutes les articulations (2, 4, 6) de la cinématique présentent un verrouillage pour le blocage, en particulier mécanique et/ou manuel et/ou actionné, de l'articulation respective, en particulier conjointement avec et/ou indépendamment d'au moins une autre articulation de la cinématique.

9. Dispositif de positionnement selon l'une quelconque des revendications précédentes, **caractérisé par** un moyen de serrage (8 ; 8'), en particulier mécanique et/ou pneumatique, qui enchaîne l'un à l'autre deux maillons (1, 3 ; 1, 35) reliés l'un à l'autre par au moins une articulation (2 ; 24, 6) de la cinématique du dispositif de positionnement et est davantage serré par un mouvement du dispositif de positionnement dans une position, dans laquelle le dispositif de positionnement présente une énergie potentielle inférieure.

10. Dispositif de positionnement selon l'une quelconque des revendications précédentes, **caractérisé en ce que** le dispositif de positionnement présente au moins deux maillons (3, 5), qui sont reliés l'un à l'autre par au moins une articulation (4) de la cinématique et peuvent être repliés dans une position de stockage, dans laquelle les deux maillons sont parallèles l'un à l'autre, au moins sensiblement, en sens inverse.

11. Dispositif de positionnement selon l'une quelconque des revendications précédentes, **caractérisé par** un support (9), au niveau duquel la base (1) est logée sur un ou plusieurs axes de manière linéairement mobile et/ou rotative.

12. Agencement de robot, avec :
un robot (10) avec un effecteur terminal (13), en particulier chirurgical, et un dispositif de positionnement selon l'une quelconque des revendications précédentes, dans lequel le robot est fixé sur la bride (7), en particulier de manière amovible.

13. Agencement de robot selon la revendication précédente, **caractérisé en ce que** le robot est fixé sur la bride (7) de manière rigide ou linéairement mobile (17) et/ou rotative (17').

14. Procédé de positionnement du robot (10) d'un agencement de robot avec un robot avec un effecteur terminal (13), en particulier chirurgical, et un dispositif de positionnement selon l'une quelconque des revendications précédentes 1 à 11, dans lequel le robot est fixé sur la bride (7), en particulier de manière amovible, est fixé sur la bride (7) en particulier de manière rigide ou linéairement mobile (17) et/ou rotative (17'), **caractérisé en ce que** le robot est commandé pour le réglage, en particulier manuel, du dispositif de positionnement dans une position de positionnement prédéfinie, en particulier sélectionnée à partir de plusieurs positions prédéfinies, et/ou après une fixation du dispositif de positionnement, un moyen de calibrage avec un point de référence fixe par rapport au robot est approché dans au moins une position du robot.
